# EUROPEAN PATENT APPLICATION

(11) **EP 1 211 505 A1**
(43) Date of publication of application: **05.06.2002**
(21) Application number: 00946365.4
(22) Date of filing: 17.07.2000
(51) Int. Cl.: G01N 25/16

(54) **METHOD FOR MEASURING SUBSTANCE AND MEASUREMENT REAGENT TO BE USED IN THE METHOD**

(30) Priority: 16.07.1999 JP 20340699
(71) Applicant: Asahi Kasei Kabushiki Kaisha, Osaka-shi, Osaka 530-8205 (JP)
(72) Inventor: KITAGUCHI, Nobuya, Fuji-shi, Shizuoka 417-0001 (JP); KANAGAWA, Akitaka, Fuji-shi, Shizuoka 416-0945 (JP); KATAKURA, Naoko, Koga-shi, Fukuoka 811-3113 (JP)
(74) Representative: Schmidtchen, Jürgen Christian
(86) International application number: JP0004792
(87) International publication number: WO0106243

(57) **Abstract**

The method for measuring substances of this invention carries out both sample preparation and detection of substances in a sample, in accordance with the photothermal conversion detection method, in a capillary of a microchip, whereby the quantity of substances, such as hemoglobin and ALP, can be measured from a very small amount of sample obtained from the constituents of living organism simply and easily and for a very short period time. In addition, the method allows the wastes caused by the measurements to be small.

Further, the method of this invention employs laser light having a long wave length as excitation light, whereby the photothermal conversion detection device can be manufactured and the measurements can be carried out at low costs.

Thus, the method for measuring substances of this invention can be suitably applied to the POC analyses and the like.

Further, the method for measuring substances of this invention allows even a blood sample having chyle therein to be measured simply and easily in accordance with the photothermal detection method.

Still further, the use of the measuring reagent of this invention allows the quantity of substances, such as hemoglobin and ALP, in a very small amount of sample obtained from the constituent of living organism to be measured stably in accordance with the photothermal converting detection method.

## Description

### Technical Field

This invention relates to a method for measuring substances in a very small amount of collected sample simply and easily. In addition, it relates to a reagent used in the above method.

### Background Art

Recently the importance has been noted of carrying out analyses and measurements right at the site or in the vicinity of the site where analyses and measurements are required (hereinafter referred to as "POC (point of care) analyses and the like") , such as analyses for bedside diagnosis which carry out measurements necessary for a medical diagnosis at the patient's side (POC analyses), analyses of hazardous chemical substances in rivers or wastes carried out at the sites, that is, on rivers or waste treatment centers, and tests for contamination carried out at the site of, for example, preparing or cooking foods, harvesting crops, or importing foods. And the development of the methods and apparatus for measurement suitable for these POC analyses and the like is being considered to be of major importance.

In these POC analyses and the like, measurements have been required to be carried out inexpensively, simply and easily, and for a short period of time. For the analyses for a medical diagnosis in particular, there have been demands for shortening of the analysis time, and decreasing of the amount of sample (blood etc.) needed for an analysis and the amount of wastes after measurements.

As amethod of measurement for use in the above POC analyses and the like, absorption spectrophotometry is usually and frequently adopted. The standard optical path length at the time of absorbance measurement is considered to be 1 cm in terms of the sensitivity of measurement; however, even if it is shortened to several millimeters, measurements are possible. Nevertheless, the sensitivity will be somewhat lowered.

As to the absorbance measurement at such lowered optical path lengths, there are disclosed methods in, for example, Japanese Patent Publication No. 10-501340, Japanese Patent Publication No. 9-504732 (these two filed by Abaxis Co., Ltd.), Japanese Patent Publication No. 9-196920 (Immunology), Japanese Patent Publication No. 8-114539 (Biochemistry), Japanese Patent Publication No. 9-196739 (Dissolving solution head sensing), Japanese Patent Publication No. 9-138195 (Analysis by the measurement of light transmitted by porous materials) (these four filed by Nihon Mediphisics Co., Ltd.).

However, if the amount of collected sample is much smaller, the volume of a detection cuvet (cell) having an optical path length in millimeters is too large for the sample's amount. And when adjusting the amount of collected sample to the volume of the cell, the concentration of the substance in the adjusted sample sometimes becomes too low to be analyzed.

In order to overcome such a problem, there have been proposed concepts of analytical method generically called µTAS (micro total analysis system) or Lab-on-Chip (Sensors and Actuators, B1 (1990), 244-248, A. Manz et al.). These methods aim at miniaturizing the apparatus for analysis in current use and carrying out microanalyses more simply and easily by conducting isolation and reaction of a sample using a chip about a few by a few cm square in dimensions which is provided with a capillary a few to a few hundred µm in both width and depth.

As to µTAS, its investigational work is still in progress, assuming that it is a method for measurement capable of analyzing even a small amount of sample (refer to Japanese Patent Publication No. 2-245655). The µTAS conducts isolation, reaction, and moreover, measurements of a sample in a groove (capillary) a few to a few hundred µm in both width and depth which is provided on the surface of a chip of, for example, glass, silicon and organic polymer at the largest 10 by 10 cm square in dimensions, or a few by a few cm square or smaller in dimensions. The use of such a microchip offers the advantages of allowing the amount of any one of collected sample, reagent required in analysis, and wastes or liquid wastes of, for example, expendables used in analysis to be small and also allowing the amount of time required in analysis to be generally small.

However, when applying the above mentioned microchip provided with a groove to the absorption spectrophotometry, since the microchip limits the optical path length applicable to about a few µm to a few hundred µm, measurements cannot frequently be carried out satisfactorily. The reason is that the absorption spectrophotometry, in principle, detects the ratio of the intensity of incident light to transmitted light, therefore, longer optical path length is required so as to obtain results with a high accuracy.

Thus, methods have been designed to irradiate the groove provided in a microchip with light not perpendicularly to the direction in which a sample liquid flows (longitudinally in the groove), but along the direction in which a sample liquid flows (typical examples of absorption spectrophotometry carried out in a groove include, for example, Japanese Patent Publication No. 8-304339); however, those methods have the disadvantages in that in the groove (capillary) formed on a plane chip, detection cannot be carried out easily in the direction of liquid flow and, in order to obtain a long optical path, the structure of the microchip as well as the detection portion becomes complicated.

As a method for detecting trace components, fluorescence spectrometry and emission spectrometry are also known. In such methods, however, the reaction for detection becomes extremely complicated because an antibody and the like labeled with an enzyme or a fluorescent dye must be attached directly of indirectly to the substance (hemoglobin etc.) as a subject of measurement in a sample for measurement.

As another method for detecting substances in a very small amount of collected sample, there has been known for some time a photothermal converting detection method (thermal lens detection method) in which the sample is excited by excitation light to form what is called a thermal lens and the changes in the thermallens are measured with probe light. For example, there are disclosed methods in Japanese Patent Publication No. S60-174933, A. C. Boccara et al., Appl. Phys. Lett. 36, 130, 1980, J. Liquid Chromatography 12, 2575-2585 (1989), Japanese Patent Publication No. 10-142177 (Molecular BioPhotonics), and Japanese Patent Publication No. 4-369467 (Yokogawa Electric Corporation).

First, the principle of the photothermal converting detection method (thermal lens detection method) will be described which utilizes a thermal lens formed by the photothermal converting phenomenon.

The sample for measurement is irradiated with light having a wavelength which is absorbed by the substance as a subject of measurement dissolved in a sample solution. As a result, the substance as a subject of measurement is excited by the excitation light and generates heat (photothermal converting effect). In this operation, it is important to select a wavelength of the excitation light in such a manner that impurities existing in the solution other than the substance as a subject of measurement do not absorb the excitation light.

The heat thus generated is transmitted to the solvent in the vicinity of the portion irradiated with the excitation light, causing local changes not only in density but also in refractive index. Thus, in the presence of the substance which absorbs the excitation light, it seems as if a concave lens were formed at the portion irradiated with the excitation light. The portion which seems to be a concave lens is then irradiated with probe light having a wavelength different from that of the excitation light. The probe light is refracted when it passes the thermal lens, therefore, the probe light to be captured by a photo-receptor undergoes a reduction in quantity due to the formation of the thermal lens.

The degree of the photothermal converting effect varies depending on the concentration of the substance as a subject of measurement; therefore, the substance as a subject of measurement can be quantitatively determined by measuring the degree to which the above light undergoes a reduction in quantity. In actuality, in order to improve signal to noise ratio, it is common that the excitation light undergoes chopping and the changes in quantity of the probe light synchronized with the frequency only are detected with a lock-in amplifier.

The thermal lens detection method usually requires two types of light sources (laser etc.): of excitation light and of probe light, as described above. There are cases where the photothermal converting detection method (thermal lens detection methods) utilizing an Ar laser and a He-Ne laser is applied to apparatus for capillary analysis in which a sample solution is supplied from the outside of a chip provided with a capillary (Bunseki No. 4, 280-284, 1997, M. Harada, et al., Anal. Chem. Vol. 65, 2938-2940, 1993, Kawanishi et al., the Analytical Chemistry Society of Japan, the Gist of Lectures at the 44^{th} Annual Meeting, p. 119, 1995).

Quantitative determination of substances, as a subject of measurement in a sample, which uses such a thermal lens detection method has been investigated.

For example, K. Sato, T. Kitamori et al. have made measurements for sunset yellow, which is a dye, by the thermal lens detection method using Ar laser light (wavelength: 488 nm) as excitation light and He-Ne laser light (wavelength: 632.8nm) as probe light (Analytical Sciences Vol .15, 525-529, 1999).

The thermal lens detection method having been improved by Kitamori et al. is such that in order to make measurements for a trace of substance in a capillary about several tens µm in both depth and width which is provided in a microchip, excitation light and probe light are condensed into the capillary utilizing a microscope. The thermal lens used in such a method may be referred to as "microscopic thermal lens".

In the use of such a microscopic thermal lens, excitation light and probe light are focused on a very small space in the capillary; therefore, a laser which creates no chromatic aberration and provides a uniform wavelength is preferably used as a light source.

There are cases where the level of β-carotene has been measured by the thermal lens detection method using a Nd : YAG laser light (wavelength: 355 nm) as excitation light and He-Ne laser light (wavelength: 632.8 nm) as probe light (for example, S. Luterotti, etal., J. Pharmaceutical and Biomedical Analysis, 21(1999), 901-909 and J. Amador-Hernandez, et al., Applied Spectroscopy Vol. 52, 1465-1471, 1998).

Further, there are cases where measurements have been made for clenbuterol useful as a pharmaceutical product by the thermal lens detection method using an Ar laser light (wavelength: 514.5 nm) as excitation light and He-Ne laser light (wavelength: 632.8 nm) as probe light (for example, Y. Martin Biosca, et al., J. Pharmaceutical and Biomedical Analysis, 41(1996), 1037-104 etc.).

In the above described thermal lens detection methods, measurements have been made utilizing light absorption of the substances as a subject of measurement in a sample in themselves and using excitation light sources which output light having a wavelength suitable for the absorption by the respective substances. However, the substance as a subject of measurement does not always have an absorption band at a wavelength convenient for measurements.

Generally, gas lasers such as Ar laser and He-Ne laser are expensive; therefore, for simple and easy POC analyses and the like, inexpensively available semiconductor lasers are desirably used as a light source. The semiconductor lasers inexpensively available at present output light generally having a wavelength of 580 nm or more, and more inexpensive ones output light having a wavelength of 600 nm or more, and much more inexpensive ones light having a wavelength of 630 nm or more.

Accordingly, for the substances as a subject of measurement which have an absorption band at a wavelength less than the above described ones, a method is required in which the substances as a subject of measurement are allowed to produce a substance different from themselves which has an absorption band at a wavelength of 580 nm or more (hereinafter referred to as substance having an absorption band at a long wavelength) by some means and the quantity of the substances as a subject of measurement is indirectly measured through measuring the quantity of the substance having an absorption band at a long wavelength.

The applicants of this invention also have filed patent applications on the method of making measurements for substances using the thermal lens detection method: Japanese Patent Application No. 10-181586 ("Apparatus and Method for Mixed Analysis"); Japanese Patent Application No. 10-181587 ("Apparatus for Capillary Photothermal Converting Analysis"); Japanese Patent Application No. 10-167603 ("Apparatus for Analysis"); International Patent Application PCT/JP99/03158 ("Apparatus for Photothermal Converting Spectrochemical Analysis"); and Japanese Patent Application No. 12-2057.

In the above specifications, methods for measuring the blood levels of total cholesterol and glutamic-oxaloacetic transaminase (GOT) are described as examples of methods for analyzing components in a very small amount of blood sample. The point of all these methods is that hydrogen peroxide is generated by the enzymatic reaction using the substances as a subject of measurement as starting materials.

Of various types of absorption spectrophotometry, the method is known in which a substance having an absorption band at a long wavelength is produced through generating hydrogen peroxide and the quantity of the substances as a subject of measurement is measured indirectly through measuring the quantity of the substance having an absorption band at a long wavelength.

For example, in the measurement of the blood level of total cholesterol, hydrogen peroxide is generated in the following steps of: obtaining cholesterol by hydrolyzing cholesterol ester with cholesterol esterase; and oxidizing the obtained cholesterol together with the cholesterol liberated in the blood from the beginning.

Then, with the hydrogen peroxide, 3,5-dimethoxy-N-ethyl-N-(2'-hydroxy-3'-sulfopropyl) anilin e sodium (hereinafter referred to as DAOS) and 4-aminoantipyrine (hereinafter referred to as 4-AA) are subjected to oxidation condensation to give a blue dye as a reaction product. Then the level of total cholesterol in blood is obtained through measuring the absorbance (wavelength: 600 nm) of the blue dye. This method of measurement is described in the explanatory pamphlet attached to HA Test Wako for Measuring Total Cholesterol and Cholesterol E - HA Test Wako manufactured by Wako Pure Chemical Industries, Ltd.

A case where the level of total cholesterol in blood is determined by the absorption spectrophotometry using N-ethyl-N-(3-methylphenyl)-N'-succinyl ethylenediamine (hereinafter referred to as EMSE) instead of DAOS is described in Izumi Kanai, An Outline of Clinical Laboratory Test (Kanahara Publishing Company, 1998, 555-556).

In the measurement of the blood level of GOT, hydrogen peroxide is produced in the following steps of: producing oxalacetate from sodium L-aspartate and α-ketoglutaric acid as substrates with GOT in a collected sample; deriving pyruvate from the oxalacetate with oxalacetate oxidase; and oxidizing the pyruvate with oxygen and pyruvate oxidase.

Then, with the produced hydrogen peroxide and peroxidase, 4-AA and N-ethyl-N-(2-hydroxy-3-sulfopropyl)-m-toluidine sodium (hereinafter referred to as TOOS) are subjected to oxidation condensation to give a dye. The level of GOT is obtained through measuring the absorbance (wavelength: 555 nm) of the dye. This method of measurement is described Collection of Package Inserts for In Vitro Diagnostic by Kainos Co., Ltd., 1998 edition, 279-280.

As described above, if hydrogen peroxide can be produced by selecting oxidase according to the substance as a subject of measurement and allowing the selected oxidase to act on the substance, an oxidation condensation product of DAOS (or similar dyes such as EMSE and TOOS) and 4-AA can be produced relatively easily. And measurements can be made for the substance indirectly through measuring the oxidation condensation product (blue dye etc.) by the thermal lens detection method using a long-wave-length laser light as excitation light.

However, it goes without saying that hydrogen peroxide cannot be derived from all the substances.

In the blood analysis useful for clinical diagnoses, for example, the substances as a subject of measurement are hemoglobin, alkaline phosphatase, etc. However, the reaction to generate hydrogen peroxide from hemoglobin, alkaline phosphatase, etc. is not common. Therefore, the above described methods of measurement utilizing the reaction which involves the production of hydrogen peroxide have never been carried out for the substances such as hemoglobin and alkaline phosphatase.

Accordingly, for the substances, such as hemoglobin, from which hydrogen peroxide is hard to derive, it is necessary to find a reagent or reaction which allows producing of a substance having an absorption band at a long wavelength from hemoglobin etc.

When carrying out measurements in a capillary, the formation of precipitates affects the measurements adversely and greatly; therefore, it is necessary to find a reagent or reaction which forms neither precipitates nor crystallites.

Then the conventional methods for making measurements for these substances will be described in detail. First, the method for quantitatively measuring hemoglobin will be described.

Measuring the level of hemoglobin in a blood sample is important in a clinical diagnosis of anemia, polyemia, etc.

The conventional methods for measuring the blood level of hemoglobin using the absorption spectrophotometry have all been improved so as to adapt them to relatively large automated testing instruments, that is, desk top ones or bench top ones. Therefore, the cuvets (cells) for measurements used are as large as about a few hundred µl to 1 ml, and the optical path length can be set at about 2 to 10 mm, which allows the detection of hemoglobin to be carried out by the absorption spectrophotometry.

The conventional methods for measuring the level of hemoglobin include, for example, cyanmethemoglobin method, SLS method, azaidomethemoglobin method and alkaline hematin method, all of which carry out the measurement by the absorption spectrophotometry.

As a method in which the whole blood is hemolyzed and the level of hemoglobin is measured by the absorption spectrophotometry, the cyanmethemoglobin method has been most frequently used, and it has been designated as an international standard. The determination of the level of hemoglobin in accordance with the cyanmethemoglobin method will be briefly described below.

First, an erythrocatalyzing agent containing a nonion surfactant etc. is added to a blood sample and the hemoglobin contained in red blood cells is eluted. Then the eluted hemoglobin is oxidized with an oxidizing agent, such as potassium ferricyanide, to be converted into methemoglobin. This methemoglobin is in turn converted into cyanmethemoglobin by adding cyan ion thereto, to prepare a stable sample for quantitative determination of hemoglobin.

The cyanmethemoglobin has an absorption band at a wavelength of 540 nm, and therefore, hemoglobin can be quantitatively determined by measuring the absorbance of the cyanmethemoglobin at a wavelength of 540 nm. The cyanmethemoglobin has an absorption band at a wavelength of 540 nm, however, does not have an absorption band at a wavelength of 600 nm or more. Accordingly, in light of the fact that the semiconductor lasers inexpensively available at present output light having a wavelength of 600 nm or more, the cyanmethemoglobin method cannot be applied to the method for quantitatively determining hemoglobin which is to use an inexpensive semiconductor laser as excitation light source or absorption light source.

In addition, since the cyanmethemoglobin method uses a measurement reagent containing potassium cyanide, a poisonous substance, there are hazards connected with handling such a measurement reagent. Furthermore, the cyanide contained in the waste liquid produced by the measurement must be decomposed with sodium hypochlorite etc. before the waste liquid is disposed of; thus, the waste disposal is troublesome.

As means for solving such a problem as is related to poisonous substances, a hemoglobin measurement reagent consisting of: a neutral buffer solution; and sodium dodecyl sulfate or sodium lauryl sulfate (hereinafter referred to as SLS), which is a anionic surfactant, and Triton X-100 (brand name) contained in the buffer solution is disclosed in Clinical Biochemistry vol. 15, 1982, 83.

In the method which uses the above reagent, first red blood cells are hemolyzed by the action of SLS and Triton X-100, and then the eluted hemoglobin is converted into methemoglobin and then into SLS hemoglobin by the action of SLS and oxygen. This SLS hemoglobin absorbs light having a wavelength of 540 nm, and therefore, hemoglobin can be quantitatively determined by measuring the absorbance of the SLS hemoglobin at a wavelength of 540 nm.

It is certain that this method does not require troublesome waste liquid disposal, since the measurement reagent used contains no cyanide. However, SLS hemoglobin absorbs light having a wavelength of 540 nm, just like cyanmethemoglobin, but does not absorb light having a wavelength of 600 nm or more. Thus, this method cannot also be applied to the optical method for quantitatively determining hemoglobin which is to use an inexpensive semiconductor laser outputting light having a wavelength of 600 nm or more as a light source.

A method for quantitatively determining hemoglobin is also known which utilizes the peroxidase activity of hemoglobin itself. Japanese Patent Publication No. S59-222766 (Terumo Corporation) discloses a method for quantitatively determining hemoglobin in the steps of: oxidizing tetramethylbenzidine with hydrogen peroxide added as a reagent by utilizing the peroxidase activity of the hemoglobin itself; and measuring the absorbance of the oxidation product (wavelength: 660 nm) in acetic acid.

Further, Japanese Patent Publication No. 2-122267 (The Green Cross Corporation) discloses amethod for quantitatively determining hemoglobin in the steps of: oxidizing tetramethylbenzidine in a citric acid solution with strontium peroxide by utilizing the peroxidase activity of the hemoglobin itself; and measuring the absorbance of the oxidation product (wavelength: 650 nm).

Any one of these methods does not include a step of hemolyzing redblood cells; therefore, the hemoglobin measured by these methods is a trace of solubilized hemoglobin contained in blood plasma, serum and urinary (about a hundredth as low as the level of hemoglobin in the whole blood (10 and several g/dl)), but not the whole hemoglobin. Further, in these methods, a calibration curve is difficult to prepare due to the hemoglobin peroxidase activity inhibiting factors existing in blood plasma etc. Still further, since these methods use the absorption spectrophotometry, they are not suitable for the measurement for a very small amount of sample using a microchip.

On the other hand, Japanese Patent Publication No. 9-278786 (Pola Chemical Industries Inc.) discloses a method, which is an improvement of the cyanmethemoglobin method which normally measures the absorbance at a wavelength of 540 nm, in which the absorbance of cyanmethemoglobin is increased by adding pectin or oligogalacturonic acid and the sensitivity of measurement is improved. In this method, while the absorbance is increased, the light which cyanmethemoglobin absorbs is shifted to light having a longer wavelength; as a result, measurement is made possible at wavelengths of 680 to 685 nm. However, even though the absorbance is increased, there is a sensitivity limit (limit of sample amount) to the absorbance measurement of this method, and moreover, the method has a problem of using cyanogens.

There are also known cases where production of abnormal hemoglobin in leukemia white blood cells is detected using the thermal lens detection method (Wu, Kitamori and Sawada, Ana. Chem. 63, 217-219, 1991, Kitamori and Sawada, Bunseki 3, 178 - 187, 1994). In this method, a single white blood cell is isolated on a slide glass under a microscope, and hemoglobin is excited with a Xe lamp to absorb light having a wavelength of 420 nm without disrupting the cell. And the level of hemoglobin produced by the white cell, which has been made abnormal due to leukemia, is measured through detecting the thermal lens formed in the upper space outside the white cell.

However, in order to determine the blood level of hemoglobin, it is necessary to hemolyze a certain volume of blood and make quantitative measurements for hemoglobin originated from all the red blood cells existing in the hemolyzed blood; therefore, this method cannot be applied to the quantitative measurements for red blood cell hemoglobin inblood which are required for a diagnosis of anemia. Moreover, since the wavelength of the excitation light used is 420 nm, it is difficult to use an inexpensive semiconductor laser.

There is also known a case where measurements are made for the hemoglobin isolated by the gel electrophoresis using the single beam as thermal lens detection method. (MA Thompson, et al., Applied Spectroscopy, Vol. 46, 945-947, 1992). Even in this case, however, an expensive Ar laser (wavelength: 514.5 nm) is used as an excitation light source. Furthermore, a large slab gel, or solid phase 18 cm×16 cm×750 µm in size is used in the measurements, and it is far from measuring a liquid sample in a microchip.

As described so far, there has been known no method for measuring the level of hemoglobin in the whole blood using a very small amount of blood sample and laser light having a wavelength of 600 nm or more.

Then, methods for making measurements for phosphatase in a blood sample will be described. As a method for determining the level of phosphatase in a blood sample, the one using p-nitrophenylphosphoric ester as a substrate is widely used as a standard method (Izumi Kanai, An Outline of Clinical Laboratory Test, Kanahara Publishing Company, 1998, 613-615). In this method, the level of phosphatase is determined in the steps of: hydrolyzing p-nitrophenyl phosphate with phosphatase to produce p-nitrophenol; and measuring the absorbance at a wavelength of 405 nm of the produced p-nitrophenol.

Since p-nitrophenol does not substantially have an absorption band at a wavelength of 550 nm or more, this reaction system cannot be adopted for the thermal lens detecting method using an in expensive laser. This is described in the package inserts for ALP-S reagent manufactured by Denka Seiken Co., Ltd..

There has been also known a method in which the level of phosphatase is determined colorimetrically in the steps of: forming red quinone dye by subjecting the produced p-nitrophenol and 4-AA to oxidation condensation with sodium metaperiodate; and measuring the absorbance at a wavelength of 500 nm of the formed red quinone dye (Collection of Package Inserts for In Vitro Diagnostic by Kainos Co., Ltd., 1998 edition, 277-278).

Further, a method has been investigated in which azo dye is used as a substrate for alkaline phosphatase (hereinafter referred to as ALP) which absorbs light having a longer wavelength, as disclosed in Japanese Patent Publication No. 7-33789 (Fuji Photo Film Co., Ltd.). This method is, however, for making measurements by the absorption spectrophotometry, not for making measurements by the photothermal converting detection method.

Common problems arising when measuring samples obtained from blood by the absorption spectrophotometry include, for example, chyle. Chyle is a white or light yellow turbid liquid introduced from the lacteal of bowels during the process of digestion, and it is carried by lymph glands into circulatory organs via a thoratic duct.

Chyle looks like milk due to chylomicron in lymph fluid (Stedmans, Medical Dictionary, Medical View Co., Ltd.). In other words, chyle is the micelle or liposome in a white turbid state formed of cholesterol or neutral fat in blood.

In a blood sample where chyle exists, "turbidity" occurs, which adversely affects measured results when the measurements are made by the absorption spectrophotometry. In other words, in the absorption spectrophotometry, it is difficult to distinguish the absorption by the turbidity from the absorption by the substances as a subject of measurement or the reaction products. Therefore, usually a control absorbance is measured with light having a longer wavelength of 700 to 800 nm at which the substances as a subject of measurement and the reaction products do not have an absorption band, and then the absorbance at a wavelength at which the substances as a subject of measurement and the reaction products do have an absorption band. The measurements are expressed by the difference between the two measured absorbance values (refer to the aforementioned cited documents, An Outline of Clinical Laboratory Test and Collection of Package Inserts for In Vitro Diagnostic by Kainos Co., Ltd., 1998 edition).

Such an operation can correct the effect of impurities and dirt in the cell (cuvet) of an absorption spectrophotometer, however, it requires measurements at two different wavelengths for each sample.

This invention has been made in order to overcome the problems attendant to the prior art described above; accordingly, its principal object is to provide a method of measurement suitably applied to POC analyses and the like, the method being characterized in that it makes it possible to measure substances in a very small amount of sample obtained from a constituent of living organism in a capillary inexpensively, simply and easily, and for a short period of time in accordance with the thermal lens detection method which utilizes an inexpensive long-wave-length laser as an excitation light source.

An object of this invention is, in particular, to provide a method of measurement which makes it possible to measure substances, such as hemoglobin and phosphatase, from which hydrogen peroxide is hard to produce.

Another object of this invention is to provide a method of measurement which makes it possible to quantitatively determine a substance even in a chyliform blood sample at a time.

Still another object of this invention is to provide a measurement reagent which is suitably used for the above method of measurement and ensures a stable measurement of substances in a very small amount of sample obtained from a constituent of living organism.

### Disclosure of the Invention

In order to achieve the above objects, this invention is constructed as follows. The method for measuring a substance according to this invention is a method for detecting a substance in a sample obtained from a constituent of living organism or measuring a concentration of the same, characterized in that it includes a sample preparation step and a photothermal converting detection step, the sample preparation step is a step of preparing a sample for measurement in the sub-steps of: mixing and reacting the above sample obtained from a constituent of living organism with a measurement reagent; and producing a reaction product, without producing hydrogen peroxide, having an absorption peak at a wavelength different from that of the absorption peak of the above substance in an amount corresponding to that of the above substance, the photothermal converting detection step is a step of measuring a change in physical quantity of the above sample for measurement with a change in local temperature of the same which is caused by irradiating the above sample for measurement with excitation light, the above reaction product being water-soluble, the absorbance of the above reaction product in the wavelength range of the above excitation light being sufficient to cause the above temperature change.

If the above change in physical quantity is a change in refractive index, the above photothermal converting detection step makes it possible to measure a change in probe light, which is made to enter a thermal lens formed due to the above change in refractive index, caused by the above thermal lens.

Preferably the wavelength of the above excitation light is 600 nm or more.

If the above sample obtained from a constituent of living organism and the above measurement reagent are charged into amicrochip including a capillary, the above sample preparation step and the above photothermal converting detection step can be carried out in the capillary of the microchip.

The above capillary may be formed by bonding together a pair of planar members each including a groove on its surface with a surface including the groove facing inside. The above groove may be provided for at least one of the above pair of planar members.

If the above sample obtained from a constituent of living organism is a blood sample and the above substance is hemoglobin, in addition, if the above sample preparation step is a step of preparing the above sample for measurement by mixing the above blood sample with the above measurement reagent to hemolyze the blood sample, the hemoglobin in the blood sample can be determined.

In the above determination of hemoglobin, preferably the wavelength of the above excitation light is in the range of 610 nm to 650 nm and the wavelength of the above probe light is longer than that of the above excitation light.

The wavelength of the above excitation light can be in the range of 620 nm to 640 nm while allowing the wavelength of the above probe light to remain longer than that of the above excitation light.

The above measurement reagent may contain a neutral surfactant in a concentration sufficient to hemolyze red blood cells, an oxidant in a concentration sufficient to oxidize hemoglobin to give methemoglobin, and a buffer in a concentration sufficient to keep the pH value of the reagent in the range of 5 to 7.

Preferably the above measurement reagent contain no cyanide.

Preferably the mixing ratios of the above blood sample to the above measurement reagent are in the range of 1:1 to 1:250.

If the above substance is phosphatase and the above measurement reagent contains a substrate for the above phosphatase, in addition, if the above sample preparation step is a step of preparing a sample for measurement by mixing the above sample obtained from a constituent of living organism with the above measurement reagent to react the phosphatase in the above sample obtained from a constituent of living organism with the substrate in the above measurement reagent, the phosphatase in the sample obtained from a constituent of living organism can be determined.

The above substrate may have a phosphoric ester linkage.

Preferably the above substrate is any one of 5-bromo-4-chloro-3-indoxyl phosphate and the salts thereof.

The above phosphatase may be alkaline phosphatase.

Preferably the concentration of the above substrate in the above sample for measurement is in the range of 1 to 15 mM.

Preferably total time from mixing the above sample obtained from a constituent of living organism with the above measurement reagent to measuring the above change in physical quantity is in the range of 3 to 15 minutes.

Further, the method for measuring a substance according to this invention is a method for detecting a substance in a blood sample with chyle or measuring the concentration of the same, characterized in that it includes a sample preparation step of preparing a sample for measurement by mixing the above blood sample with a measurement reagent and a photothermal converting detection step of measuring a change in physical quantity of the above sample for measurement with a change in local temperature of the same which is caused by irradiating the above sample for measurement with excitation light.

If the above change in physical quantity is a change in refractive index, the above photothermal converting detection step makes it possible to measure a change in probe light, which is made to enter a thermal lens formed due to the above change in refractive index, caused by the above thermal lens.

If the above blood sample and the above measurement reagent are charged into a microchip including a capillary, the above sample preparation step and the above photothermal converting detection step can be carried out in the capillary of the microchip.

The above capillary may be formed by bonding together a pair of planar members each including a groove on its surface with the surface including the groove facing inside. The above groove may be provided for at least one of the above pair of planar members.

Further, the measurement reagent according to this invention is a measurement reagent used for the above described method for measuring a substance, in particular, hemoglobin, characterized in that it contains a neutral surfactant in a concentration sufficient to hemolyze red blood cells, an oxidant in a concentration sufficient to oxidize hemoglobin to give methemoglobin, and a buffer in a concentration sufficient to keep the pH value of the reagent in the range of 5 to 7.

Further, the measurement reagent according to this invention is ameasurement reagent used for the above described method for measuring a substance, in particular, phosphatase, characterized in that it contains a substrate having a phosphoric ester linkage.

Preferably the above substrate is any one of 5-bromo-4-chloro-3-indoxyl phosphate and the salts thereof.

In the following this invention will be described in detail.

As a method for measuring components contained in samples obtained from living organism, such as blood and urine, or in samples for environmental analyses, if the amount of the samples is very small, a method is useful which uses a microchip including a capillary and the thermal lens detection method in combination. As described in the section of Background Art, in order to measure a substance in a capillary about several tens to several hundred µm in both depth and width in accordance with the thermal detection method, preferably excitation light and probe light are condensed into the capillary.

Therefore, as light sources of the above two types of light, it is preferable not to use white-light sources, which have problems of chromatic aberration etc., but to use lasers or light emitting diodes of which wavelength is uniform. However, lasers or light emitting diodes outputting short-wave-length light are very expensive, in addition, in order to form a thermal lens, the excitation light source used is required to have a certain degree of output. Accordingly, long-wave-length semiconductor lasers, which are relatively inexpensive at present and have a certain degree of output, are preferably used. Light emitting diodes can also be used as an inexpensive light source in this invention, as long as they have output as high as about several mW or more.

The wavelengths of excitation light and probe light shall be longer than 580 nm. Semiconductor lasers and light emitting diodes outputting light having wavelengths of 600 nm or more, and moreover, 630 nm or more are particularly preferable because they are inexpensive.

With these points as background, the conditions under which a substance in a capillary is measured in accordance with the thermal lens detection method will be described below.
a) A substance as a subject of measurement itself or a substance enzymatically/chemically produced therefrom (hereinafter referred to as reaction product) has absorbance sufficient to form a thermal lens within the wavelength range of excitation light.
b) Neither substance as a subject of measurement nor reaction product forms a precipitate in the capillary.

Further, the wavelength of probe light is preferably longer than that of excitation light; this is not an essential condition, though.

In the following the above described conditions will be described in detail.

Of the substances existing in samples obtained from living organism, such as blood and urine, very few have an absorption band at a wavelength of 580 nm or more, in particular, at a wavelength of 630 nm or more. Even hemoglobin, which has an absorption band at a relatively long wavelength, has a strong absorption band in the range of 540 to 577 nm and only a weak absorption band at 630 nm, which is attributed to methemoglobin existing in living organism in a very small quantity.

Bilirubin, for example, also has a considerably weak absorption at 630 nm or more, and it is hard to quantitatively determine.

Many enzymes existing in blood are white protein normally having an absorption band only in the ultraviolet, in addition, the absorption bands of enzymes and proteins as impurities are overlapped each other, whereby they are difficult to quantitatively determine. Therefore, in the determination of the blood level of an enzyme, the following steps must be taken to determine the quantity of the enzyme (to be precise, enzymatic activity): producing a reaction product using a substrate specific for the enzyme, which is to be changed to a substance (reaction product) having absorption within a long-wave-length range by the enzyme; and quantitatively determining the reaction product. Hereinafter this type of reaction will be referred to as long-wave-length absorption producing reaction.

In the determination of the blood levels of substances other than enzymes, such as cholesterol, a reaction product having absorption in the long-wave-length region must be produced from each substance with the aid of the action or chemical reaction of an enzyme which selectively acts on the substance as a substrate (the substance react with the enzyme).

Accordingly, in order to make possible measurements using an inexpensive excitation light source, it is necessary to find a reaction or a measurement reagent which enables producing of the above described reaction product from a substance as a subject of measurement in such a manner as to allow the reaction product to have an absorption band at 580 nm or more, more inexpensively 600 nm or more, and much more inexpensively 630 nm or more. The upper limit of the wavelength at which the reaction product has an absorption band is not particularly specified as long as the reaction product absorbs a sufficient amount of excitation light and the light source is inexpensive; however, a wavelength of 800 nm or less is usually used.

One of the streams of coloring reagent development in clinical chemistry analysis is, for example, to decrease the effects of interfering substances in blood on the analysis. As means for this, coloring reagents were investigated which have absorption bands at wavelengths longer than the absorption wavelengths inherent in the constituents of living organism. Even now, the development is continued of coloring reagents having λₘₐₓ longer than the absorption wavelengths of hemoglobin in hemolyzed blood samples and bilirubin in samples obtained from patients suffering from jaundice (Norihito Aoyama, Clinical Laboratory Test, 41, 9, 1014 (1997)). In order to decrease the effects of hemoglobin and bilirubin in blood, measurements must be made within the wavelength range of at least 600 nm or more (reference data in package inserts of Interference Check/A Plus (International Reagents Corporation)). As to determination of hemoglobin, when measuring hemoglobin and other biochemical items with a common light source, the wavelength of the light source in the range of 600 to 650 nm is important, since methemoglobin has an absorption band at 630 nm in acid atmospheres.

Formation of precipitates in the above described long-wave-length absorption producing reaction is undesirable, since mixing, reaction and measurements are carried out in a capillary of a microchip. Formation of so large precipitates as to be an obstacle to the flow of liquid (sample for measurement) must be avoided within the capillary or in the portions other than the capillary of a microchip. Even a slight disorder of the liquid flow may affect adversely the control of quantitative mixing and reaction time.

Precipitates formed on the detection portion of a microchip is very likely to lower the reliability or the precision of measurements, because they cause scattering of excitation light and probe light. Furthermore, the inventors of this invention have found that even very small deposits (fine precipitates, crystallites) of the reaction product of long-wave-length absorption producing reaction, which is almost no problem in the absorption spectrophotometry, is a significant problem in the thermal lens detection method.

As an example, the measurement on ALP will be described. The quantitative determination of ALP is generally carried out through measuring the activity of ALP. In other words, when ALP hydrolyzes the phosphoric ester portion of a substrate, a pigment originated from the substrate produces color; therefore, ALP can be determined by measuring the pigment.

Usually, p-nitrophenol phospate is used as the substrate of ALP, and the absorption of p-nitrophenol as the hydrolysis product at around 405 nm is measured with an absorption spectrophotometer. However, when using an inexpensive excitation light source, it is necessary to produce a reaction product having an absorption band at a longer wavelength as described above. Study reagents commercially available as substrates for ALP and capable of producing a reaction product having an absorption band at around 630 nm include, for example, 5-bromo-4-chloro-3-indoxyl phosphate.

The hydrolysis product of this substrate, 5-bromo-4-chloro-3-hydroxyindole, undergoes oxidation by oxygen and dimerization, yielding a blue pigment having an absorption band at 600 nm or more. Since this dimmer is not highly water-soluble, there should exist a concentration range suitable for the thermal lens detection method in the reaction product of the long-wave-length absorption producing reaction produced under such conditions as is a combination of ALP concentration, substrate concentration, reaction time, etc. If the concentration of the reaction product is higher than the above range, crystallites may be formed as in a case of a comparative example to be described later and thereby ALP is difficult to measure in accordance with the thermal detection method.

Such crystallites are suspended and dispersed in a solution in normal handling and are never precipitated even after standing overnight. Although these crystallites have little adverse effect on measurements in accordance with the absorption spectrophotometry, they sometimes have adverse effect on measurements in accordance with the thermal lens detection method. Accordingly, they must be precipitated by, for example, intensive centrifugation.

The reasons the output (thermal lens signal) in accordance with the thermal lens detection method becomes instable when crystallites (fine precipitates) absorbing excitation light are suspended and dispersed in a solution have not be theoretically clarified yet. Even if latex beads (about 500 nm in diameter) of polystyrene etc., which are also fine particles but do not absorb excitation light, exist in a solution, the output in accordance with the thermal lens detection method can be measured.

The fact that even if turbidity or fine particles which do not absorb excitation light exist, the output in accordance with the thermal lens detection method can be measured is of advantage particularly when measuring substances in a blood sample where chyle exists.

As described in the section of Background Art, the existence of chyle in a blood sample adversely affects measurement of substance's absorbance over a wide range of wavelength. Therefore, it is indispensable to make measurements at two different wavelengths: at an absorption wavelength of the substance as a subject of measurement; and at a longer wavelength at which turbidity is measured to examine the effects of chyle.

On the other hand, in the thermal lens detection method, if a proper dilution is made, which will be described later, the existence of chyle have little adverse effect on measurements. This is because the output in accordance with the thermal lens detection method is detected by chopping (on/off) excitation light with a frequency of about 100 to 3000 Hz and picking up only the change in quantity of probe light synchronized with the chopped excitation light. Turbidity absorbing no excitation light makes the absolute value of absorbance seem to be large without the change with time, but it does not cause thermal changes synchronized with the chopped excitation light in the sample for measurement.

In order to measure hemoglobin in accordance with the thermal lens detection method, it is necessary to find a method (measurement reagent, reaction) for producing hydrogen peroxide from hemoglobin and then producing a substance having an absorption band at a long wavelength with the aid of the hydrogen peroxide. In other words, it is necessary to find an oxidase (producing hydrogen peroxide) specific for hemoglobin or improve the reaction system so as to allow the peroxidase activity of hemoglobin itself, which is described in the section of Background Art, to be applied to the measurement of hemoglobin in the whole blood. If such a method can be found, an oxidation condensation product (a substance having an absorption band at a long wavelength) of DAOS and 4-AA can be produced with the aid of hydrogen peroxide and peroxidase.

However, any one of the above methods has a problem that oxidase is difficult to obtain or peroxidase activity of hemoglobin in the whole blood is difficult to utilize for quantitative determination. Consequently, it is suitable to use a substance having an absorption band at a long wavelength, such as one having hemoglobin itself, that is, to use methemogobin when using an inexpensive excitation light source.

Since methemogobin is highly water-soluble, it does not have a problem of forming a precipitate during the measurement. However, it is instable, therefore its measuring method has been designed to overcome the instability, so as to enable a stable quantitative measurement.

In the following the methods for measuring hemoglobin and ALP will be described in further detail.

### (Absorption Wavelength of Hemoglobin)

Most of hemoglobin in blood exists in the form of oxyhemoglobin which is bivalent iron combined with oxygen and in the form of deoxyhemoglobin which is bivalent iron combined with no oxygen, and only a very small part of the same exists in the form of methemoglobin which is oxidized tervalent iron and cannot be combined with oxygen.

Oxyhemoglobin has absorption bands at 540 nm and 577 nm and deoxyhemoglobin has an absorption band at 555 nm. As to methemoglobin, its absorption changes depending on pH value, and it has an absorption band at 630 nm when its solution is acidic and it has an absorption band in the short wavelength range and has an increased reddish tint when its solution is strongly basic.

As described above, hemoglobin in blood, just thinking of oxyhemoglobin and deoxyhemoglobin, has at least three absorption bands; therefore, the whole hemoglobin is difficult to quantitatively determine as it is. Thus, in the prior art, the whole hemoglobin is oxidized to methemoglobin by first hemolyzing a blood sample and then adding an oxidant such as potassium ferricyanide. The methemoglobin thus produced is, however, instable, and therefore potassium cyanide (KCN) or the like is added thereto to subject CN ion to coordinate bond with heme iron, yielding a stable cyanmethemglobin, which has an absorption band at 540 nm. And then the absorbance at 540 nm is measured.

However, as described above, it is difficult to carry out a quantitative measurement for hemoglobin in a microchip in accordance with the absorption spectrophotometry, and it is preferable to carry out detection in accordance with the thermal lens detection method. Taking into consideration using the method in POC analyses and the like, it is preferable to excite hemoglobin not using an expensive gas laser, but using an inexpensive semiconductor laser so as to detect changes in the thermal lens. Since the wavelength of light output from an inexpensive semiconductor laser or light emitting diode is 600 nm or more at present, it is necessary to make the whole hemoglobin in blood a hemoglobin derivative having an absorption band at 600 nm or more.

Then the inventors of this invention examined the stability of absorption by methemoglobin being irradiated with excitation light. Specifically, methemoglobin as a sample for measurement (liquid having undergone reaction) was put into a cuvet of which optical path length was set as long as 1 cm, and its absorbance was measured at the same wavelength as that of the excitation light with which a capillary of a microchip is irradiated in the thermal lens detection method.

In principle, if absorbance is instable, output in accordance with the thermal lens detection method also should be instable; therefore, providing a stable and highly accurate measurement of absorbance is a requirement to carry out the thermal lens detection method.

As shown in the experimental example 1 described later, hemoglobin was oxidized to methemoglobin having an absorption band at 630 nm without adding any potassium cyanide. Then the methemoglobin was irradiated with laser light having a wavelength of 633 nm, and whether the change in absorbance is volume independent or not was examined. As a result, it was found that hemoglobin could be quantitatively determined using methemoglobin having an absorption band at 630 nm.

### (Blood Sample/ Measurement Reagent Mixing Ratio)

After inventors' intensive investigations, however, it was found that when preparing a sample for measurement bymixing a blood sample and a hemoglobin measurement reagent in the ordinary ratio (1 : 250 or more), the output in accordance with the thermal lens detection method is somewhat instable relative to time and decreases little by little with time. For example, when the mixing ratio is 1 : 250, the absorbance at 630 nm decreases by no more than 8.5% for 20 minutes, when 1 : 415, by 16%, and when 1 : 1250, by as much as 31%, which is too low an accuracy for the method to be applied to clinical diagnoses.

Measurements canbemade for hemoglobin even in such mixing ratios, as long as the required accuracy is relatively low; however, in order to make measurements with a high accuracy, the mixing ration must be improved. After intensive investigations, the inventors found that the change (decrease) in output in accordance with the thermal lens detection method with time can be avoided if the mixing ratio of blood sample to measuring regent, which has a composition described later, is 1: 1 to 1 : 250 or less.

Further, if the mixing ratio of blood sample to measuring regent is 1 : more than 250, for example, 1 : 500, it is difficult to mix them in a microchip in which measurements for hemoglobin are intended to make. One of the reasons is that a reservoir for measurement reagent in a microchip becomes too large, since even a very small amount of blood sample requires a large amount of measurement reagent.

Furthermore, it is difficult to uniformly mix a blood sample and a measuring regent in the ratio of 1 : 500 in a narrow capillary of a microchip. Accordingly, a special flow path structure (baffle plate, stirring, etc.) or an extremely long flow path is required to realize uniform mixing. In that respect, it is preferable to keep the mixing ratio small.

Preferably the mixing ratio of blood sample to measurement reagent according to this invention is 1 : 1 to 1 : 250, more preferably 1 : 1 to 1 : 50, and much more preferably 1 : 1 to 1 : 10. If the mixing ratio is 1 : less than 1, for example, 1 : 0.1, it is difficult to provide a measurement reagent and osmotic pressure sufficient for hemolyzing, in addition, the effects of blood viscosity on the liquid supply within the microchip cannot be ignored.

### (Composition of Measurement Reagent)

The types of neutral surfactants contained in the measurement reagent used in the hemoglobin measuring method according to this invention is not limited to any specific ones, as long as they have surface active capacity sufficient to dissolve the whole red blood cells. The types of neutral surfactants used include, for example, polyoxyethylene ethers such as 6-octylphenoxypolyethoxyethanol (for example, TritonX-100, manufactured by Sigma Co., Ltd.); and polyoxyethylene sorbitans (Tween) such as polyoxyethylene sorbitan monolaurate (for example, Tween 20 manufactured by ICICo., Ltd. and Sigma Co., Ltd.) and polyoxyethylene sorbitan monooleate (for example, Tween 80 manufactured by ICI Co., Ltd. and Sigma Co., Ltd.).

In some cases, anionic surfactants such as SLS and cationic surfactant such as cetyltributylammoniumbromiode (CTAB) can be used. These ionic surfactants can also be used to such an extent that it does not change pH value during hemolyzing. A detailed list of surfactants are described in books. For example, Handbook of Surfactant, Gower Publishing Company, Ver. 2, Vols. 1 and 2 (1997).

The measurement reagent according to this invention can be added in such an amount that hemolyzes all the red blood cells in a blood sample. The measurement reagent according to this invention is added to a blood sample in the ratio of 1 : 1 to 1 : 250. As a result, the concentration of a surfactant in the obtained mixture is preferably 0.5 to 1.1% by weight, more preferably 0.7 to 0.9% by weight and much more preferably 0.8% by weight.

The oxidants usable in the measuring agent are not limited to any specific ones, as long as they can oxidize hemoglobin to methemoglobin; however, preferably potassium ferricyanide is used. The measurement reagent according to this invention is added to a blood sample in an amount sufficient to oxidize hemoglobin.

As the results of examining the oxidant concentration is shown in the experimental example 1 described later, oxidation of hemoglobin to methemoglobin, which is a requirement to carry out the thermal lens detection method, is performed preferably by adding 50 to 10000 nmol (absolute amount) of oxidant to 1 µl of blood sample and more preferably by adding 100 to 4000 nmol (absolute amount) of oxidant to 1 µl of blood sample.

The pH value of this measurement reagent is preferably in the range of weak acid to weak base so as to keep methemoglobin having an absorption band at 630 nm. The stability of absorption of methemoglobin being irradiated with excitation light, which is a requirement to carry out the thermal lens detection method, was examined in the same manner as the experimental example 1 (refer to the experimental example 2 described later). The results show that the pH value is preferably in the range of 4 to 8, more preferably in the range of 5.0 to 7.0, and much more preferably 5.7 to 6.3. In order to maintain the above pH value, buffers in common use, such as phosphoric buffer solution (for example, PBS) and various types of Good's buffers, are used.

The requirements for the reaction system and the property of the reaction product in the method of measurement according to this invention are as follows:
c) the reaction system does not employ the hydrogen peroxide pathway;
d) the reaction product is a pigment substance having an absorption band at the wavelength of an inexpensive semiconductor laser; and
e) the reaction product is in the form of a solution having sufficient uniformity relative to the laser diameter.

Of the semiconductor lasers presently on the market, inexpensive ones have an absorption band in the wavelength range about 635 to 645 nm or more. Accordingly, the reaction system in the method of measurement according to this invention is preferably such that it produces a pigment substance having an absorption band in the above wavelength range.

### (Quantitative Determination of Alkaline Phosphatase Activity)

The methods for measuring phosphatase activity include, for example, one which measures the absorption spectrum of red quinone pigment (λₘₐₓ: 500), which is an oxidation condensation product of phenol as a decomposition product of phenylphosphoric acid and 4-AA, in the wavelength range of 600 nm or more. The methods also include one which uses glycerophosphate directly as a substrate and reduces the produced glycerol to reduced nicotinamide adenine dinucleotide (hereinafter referred to as NADH) and dihydroxycetone using glycerol dehydrogenase and oxidized nicotinamide adenine dinucleotide (hereinafter referred to as NAD+) (A. Bianchi, et al., J. Biochem. Biophys. Methods, 28, 35, 1994).

The methods using a reaction system containing glycerophosphate as a substrate include, for example, one which uses NADH and tetrazolium salt, in particular, WST series manufactured by Dojin Laboratories as a substrate and adds thereto a reaction system which reduces glycerol with phenazine metasulfate (hereinafter referred to as PMS) or diaphorase activity to produce color, so as to measure the absorption spectrum of the pigment at a wavelength of 600 nm or more.

However, for the method in which the red quinone pigment having λₘₐₓ of 500 nm is measured in the wavelength range of 600 nm or more, the resolving power is decreased in the measurement of phosphatase activity. On the other hand, using a reaction system containing glycerophosphate as a substrate is certainly considered to be effective; however, since the reaction system is amultistep reaction, it requires optimizing the combination of reaction systems of substrate etc. Thus further examination period is required to use the reaction system as a reaction system in the method of measurement according to this invention.

The substrates which are used for measurement of phosphatase activity and produce color having an absorption band in the wavelength range of 600 nm or more include, for example, one disclosed in Japanese Patent Publication No. 7-33789. This substrate is a water-soluble substrate for phosphatase having an absorption band at 644 nm and can be used as a substrate for measuring ALP in the method of measurement according to this invention; however, it is not readily available. The known commercially available substrates include, for example, 5-bromo-4-chloro-3-indoxyl phosphate (hereinafter referred to as BCIP), which is decomposed to produce color having an absorption band at 615 nm and 3-indoxyl phosphate to produce color having an absorption band at 680 nm (SUBSTRATE and Reagent, a brochure from BIOSYNTH, Co., Ltd.)

As an example showing the efficacy of the method of measurement according to this invention, the use of BCIP as a substrate for measuring ALP will be described. It is widely known that 5-bromo-4-chloro-3-indoxyl (hereinafter referred to as BCI) is used for the identification of the expression of genes etc. using ALP activity, or the detection in tissue staining (J. P. Horwitz, et al., J. Med. Chem., 9, 447, 1966). It is also known that the dimer of 5-bromo-4-chloro-3-hydroxyindole (hereinafter referred to as BCI2) is poor in water-solubility and hard to solubilize (J. J. Leary, et al., Proc. Natl. Acad. Sci., USA. 80, 4045, 1983).

Thus, it is self-evident that it is difficult to measure BCI in the liquid state in the quantitative determination of ALP activity. However, after intensive investigations, the inventors of this invention have found that optimizing the concentration of BCIP in a sample for measurement makes the method of measurement according to this invention effective in the measurement of ALP activity which is used in clinical chemistry.

In particular, the concentration and reaction time of BCIP were specified which inhibits the formation of pigment particles of BCI2 having a diameter within the range of about 0.1 to 1 µm or less.

In further particular, a buffer was prepared in accordance with JSCC common standard method. Specifically, a buffer of 1.01 M 2-ethylaminoethanol-HCl (Aldrich Co., Ltd.)/0.505 mM MgCl₂ (pH 9.87) was prepared (hereinafter referred to as buffer 1). An ALP measurement reagent (measurement reagent) was prepared right before reacting it with a blood sample by dissolving BCIP (Wako Pure Chemical Industries, Ltd.) as a substrate in the buffer 1 to give a final concentration of 1 to 15 mM.

The final concentration herein used means the BCIP concentration in a sample for measurement prepared by mixing a blood sample and a measurement reagent.

Preferably the reaction time was 3 to 15 minutes, and more preferably 3 to 6 minutes.

### (Solubility of BCIP)

The solubility of BCIP as a substrate in pure water is 20 mg/ml, that is, 54 mM when it is in the form of a sodium salt (Sigma General Brochure, SIGMA Co., Ltd.). The solubility of BCIP in the buffer 1 is about 70 mM. In order to obtain the optimum concentration of BCIP, first an ALP measurement reagent was prepared by dissolving BCIP in the buffer 1 to give a final concentration of 70 mM, and then the prepared reagent was diluted with the buffer 1 step by step to prepare ALP measurement reagents having BCIP final concentrations of 70 mM, 35 mM, 15 mM, 10 mM, 6.25 mM, 5 mM, 2.5 mM and 1.25 mm, respectively.

As an ALP sample, Switrol A (Nissui Pharmaceutical Co., Ltd.) of which preparation process was partly changed was used. In particular, an ALP sample was prepared by dissolving one vial of freeze-dried Switrol A in 2500 µl of purified water (Kyoei Seiyaku Co., Ltd.) to give ALP activity of 800 IU/l in terms of calculated value, and this was made a stock solution. Then the stock solution was diluted with purified water to prepare a solution having ALP activity of 400 IU/l and 50 IU/l in terms of calculated value.

### (Measurement of ALP Activity with Absorption Spectrophotometer)

1000 µl of ALP measurement reagent and 25 µl of the above ALP sample were fully admixed in a test tube and the admixture was kept at 37°C accurately for 2 minutes and 30 seconds. Then the whole amount of the reaction solution was put into a quartz cell having an optical path length of 1 cm, and the absorbance at 635 nm was measured with a absorption spectrophotometer air thermostated at 37°C for 12 minutes and 30 seconds after starting the reaction.

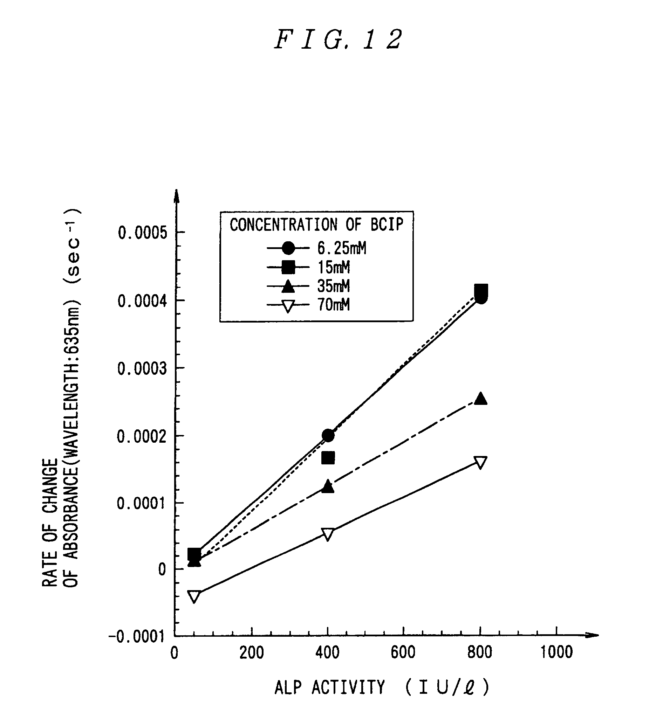

As a result, in any one of the ALP measurement reagents having respective BCIP concentrations, a calibration carve having concentration dependence was obtained, which indicated that ALP activity could be measured with an absorption spectrophotometry (refer to Figure 12).

### (Measurement of ALP activity in accordance with Thermal Lens Detection Method)

1000 µl of ALP measurement reagent and 25 µl of the above ALP sample were fully admixed in a test tube and the admixture was kept at 37°C accurately for 2 minutes and 30 seconds. Then the whole amount of the reaction solution was put into a quartz cell having an optical path length of 50 µm and the cell was placed on a stage air thermostated at 37°C, and measurement was made in accordance with the thermal lens detection method for 5 minutes after starting the reaction.

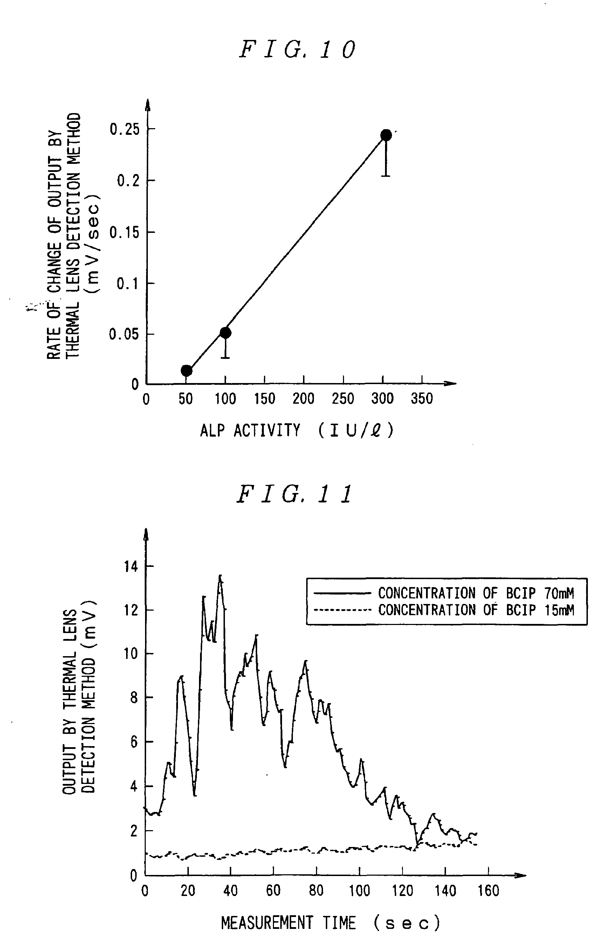

As a result, in the ALP measurement reagents having a BCIP concentration other than 70 mM and 35 mM, calibration carves having concentration dependence were obtained, which indicates that ALP activity can be measured in accordance with the thermal lens detection method (refer to Figures 10 and 11).

Thus, it was found that when using an absorption spectrophotometry, ALP activity can be measured at all the BCIP concentrations, but on the other hand, when using the thermal lens detection method, ALP activity is difficult to measure at some BCIP concentrations. On the assumption that this is due to the existence of colored fine particles (pigment fine particles), the particle size distributionof the reaction products was measured for ALP measurement reagents having respective BCIP concentrations.

### (Measurement of BCI2 Particle Diameter with Laser Diffraction/Scattering Particle Size Distribution Meter)

Twenty-five ml of each of the ALP measurement reagents having BCIP concentrations of 70 mM and 6.25 mM was preheated to 37°C. Then the mixtures (samples 1 to 4) were prepared in the manner shown below of the above ALP measurement reagents and standard serum (blood sample) prepared to have ALP activity of 800 IU/l in terms of calculated value.

First, the sample 1 was prepared by mixing the ALP measurement reagent having a BCIP concentration of 70 mM with 2500 µl of the above standard serum and allowing them to react with each other at 37°C for 3 days.

Then, the sample 2 was prepared by mixing the ALP measurement reagent having a BCIP concentration of 70 mM with 625 µl of the above standard serum and allowing them to react with each other at 37°C for 3 days.

The sample 3 was prepared by mixing the ALP measurement reagent having a BCIP concentration of 6.25 mM with 625 µl of the above standard serum and allowing them to react with each other at 37°C for 3 hours.

The sample 4 was the ALP measurement reagent having a BCIP concentration of 6.25 mM itself (not mixed with the above standard serum).

The measurement of particle size distribution was carried out with a HORIBA LA-910 type Laser Diffraction/Scattering Particle Size Distribution Meter in accordance with the manual thereof. Specifically, particle size distribution was measured in state where 10 ml of each of the samples 1 to 4 was added to about 50 ml of purified water contained in an ultrasonic dispersion bath attached to the above instrument. Relative refractive index, which is a requirement to measure particle size distribution, was set at 1.26 - 0.00i.

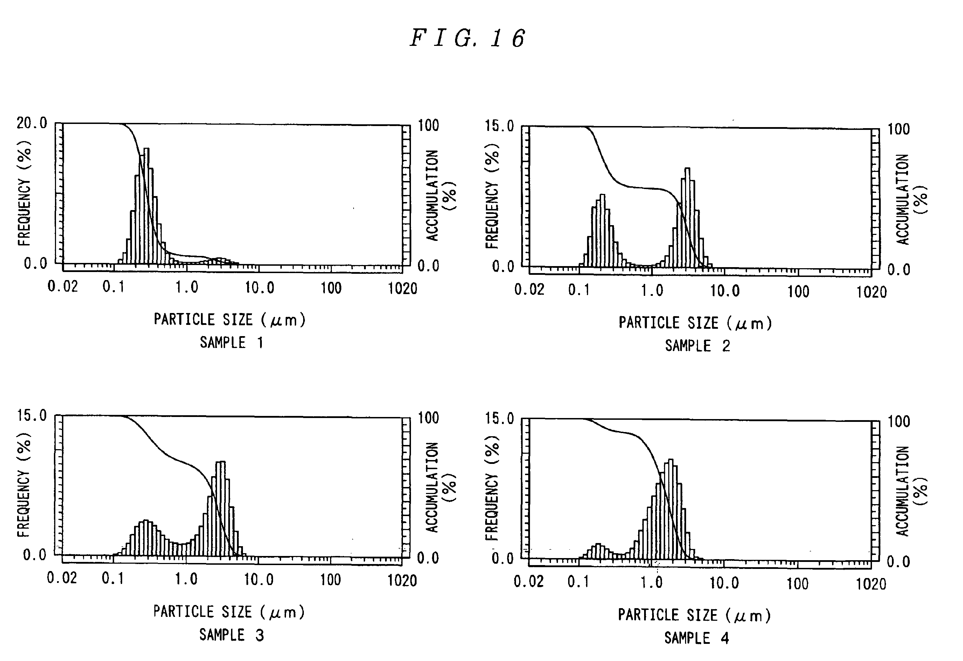

The result of the measurement revealed that there existed BCI2, which was produced through the above reactions, in some samples obtained by mixing each of the ALP measurement reagents and standard serum. It also revealed that the particles (crystallites) of BCI2 were about a few hundred nm in diameter (refer to Figure 16).

Further, the result of the particle size distribution measurement revealed that there existed particles a few µm in size. For the samples prepared through a long reaction time, in other words, for the samples 1 and 2 prepared through 3-day reaction time, since most particles existing therein were a few hundred nm in diameter, the particles a few µm in size are considered to be BCIP. It is, however, not clear in what state the BCIP particles exist.

Thus, a measurement reagent suitable for the thermal lens detection method is required not to form pigment particles as a reaction product of the measurement reagent and a sample obtained from the constituent of living organism. Further, the BCIP concentration and reaction time of the ALP measurement reagent must be selected properly.

It goes without saying that it is not desirable colored crystallites such as BCI2 exist in a measurement reagent or a sample obtained from the constituent of living organism before reacting them with each other.

### (Quantitative Determination of Lactate Dehydrogenase)

When measuring the activity of lactate dehydrogenase (hereinafter referred to as LDH), lactate and NAD+ are changed to pyruvate and NADH with LDH activity, and NADH and NTB are quantitatively converted to NAD+ and insoluble diformazan with diaphorase activity. Then the insoluble diformazan is solubilized with 0.1 N - HCl and the absorbance is measured at a wavelength of 570 nm. Phenazine metasulfate (whereinafter referred to as PMS) is sometimes used instead of diaphorase.

The absorption wavelength spectrum indicates that the solubilized pigment (insoluble diformazan) can be satisfactorily determined through measuring the absorbance in the wavelength range of 600 nm or more.

Further, a method is known in which water-soluble substrate for formazan is applied instead of NTB (Munetaka Ishiyama, Clinical Laboratory Test, 41, 9, 995, 1997). Of water-soluble Formazin manufactured by Dojin Laboratories, WST-4 and WST-5 in particular have a characteristic of showing no change in spectrum with pH value of the solution (Munetaka Ishiyama, DOJIN News, 82, 10, 1996), and though their maximum absorption wavelength is 550 nm, the absorption wavelength spectrum indicates that measurements can be made through measuring the absorbance in the wavelength range of 600 nm or more.

WST-1, WST-3 and WST-8 have absorption maxim at 438 nm, 433 m and 460 nm, respectively, and have no absorption wavelength spectrum in the range of 600 nm or more. However, increasing the pH value using an alkali after completing the above reaction allows them to have an absorption band at 600 nm or more (spectrum shift), accordingly, they can be applied to the method of measurement according to this invention.

However, if adjustment of the pH value is carried out with a strong acid or a strong base in a microchip or solubilizing of insoluble pigment with a solvent etc. is carried out in a microchip, so as to solubilize the insoluble pigment and give rise to a spectrum shift, damages such as dissolution may be caused in polymer etc. which constitutes the microchip. This is not desirable because such damages are likely to cause a change in the cross-sectional area of a capillary, and hence a change with time in the flow rate of the liquid flowing within the capillary.

Thus, WST-4 and WST-5 were applied to this quantitative determination as a water-soluble substrate. With WST-4 and WST-5, the inventors of this invention could show the effectiveness of the method of measurement according to this invention within the range of the LDH activity measured in clinical laboratory tests.

Then the effectiveness of the method of measurement according to this invention will be shown taking several examples of the reaction systems which do not employ the hydrogen peroxide pathway.

### (Determination of Blood Level of Total Protein)

The methods for determining the blood level of total protein (hereinafter referred to as TP) without employing a reaction through which hydrogen peroxide is derived from a substance as a subject for measurement include, for example, buret colorimetry. In this method, a complex of Cu ion and nitrogen atom in a peptide chain is allowed to produce color in an alkaline atmosphere and determination is made through measuring the absorbance in the wavelength range of 540 to 550 nm.

A method which uses a pyrogallol red pigment and measures the absorbance at 600 nm is also in common use (Collection of In Vitro Diagnostic, Yakuji Nippo Limited, 1997). However, the method using a pyrogallol red pigment can determine only low blood levels of total protein and the determination of TP require 1000-fold or more dilution of blood.

Quantitative dilution can be made in a microchip. In actuality, however, it is impossible in actuality to make a 1000-fold or more dilution in a microchip, because the reservoirs for dilution provided in a microchip become too large.

The inventors checked the absorption spectrum of a commercially available measurement reagent for buret colorimetry (Kainos Auto Series TP Reagent, Kainos Co., Ltd.) and found that TP can be determined by measuring the absorbance at 635 nm.

### (Determination of Blood Level of Urea Nitrogen)

The methods for determining the blood level of urea nitrogen include, for example, the diacetylmonooxime method, the urease/indophenol method, the urease/UV method, and the one in which hydrogen peroxide is produced for the purpose of quantitative determination by the enzymatic process. In general, the urease/indophenol method is most frequently used (Collection of In Vitro Diagnostic, Yakuji Nippo Limited, 1997).

The urease/indophenol method is such that it does not employ a reaction through which hydrogen peroxide is derived from a substance as a subject of measurement and carries out the determination through measuring the absorbance at 570 nm.

The inventors of this invention employed a commercially available measurement reagent for urease/indophenol method (a reagent for automated analyses, "Seiken"UN-I, Denka Seiken, Co., Ltd.) and checked the spectrum of the reagent in accordance with the urease/indophenol method, and found that the urea nitrogen can be determined through measuring the absorbance at 635 nm.

### (Determination of Blood Level of Total Bilirubin)

The methods for determining the blood level of total bilirubin in common use include, for example, the diazo method in which azobilirubin is produced by linking diazo pigment to bilirubin and the enzymatic method in which bilirubin is oxidized with bilirubin oxidase (Collection of In Vitro Diagnostic, Yakuji Nippo Limited, 1997).

However, the enzymatic method cannot be applied to the method of measurement according to this invention, since it carries out the determination making use of the decrease in a specific absorption wavelength (around 450 nm) whenbilirubin is oxidized.

The inventors of this invention confirmed that the blood level of total bilirubin can be determined through measuring the absorbance at 600 nm or more using the alkali azobilirubin method (measuring wavelength: 600 nm).

### (Determination of γ-GTP Activity)

The methods for determining the activity of γ-GTP in blood in common use include, for example, the p-nitroanilide method or carboxy-p-nitroanilide method (405 to 415 nm), 3-carboxy-4-hydroxyanilide method (410 to 635 nm), dimethylaminoanilide method or the diethylaminoanilide method (660 nm), and the γ-Glu-DBHA method (600 to 610 nm) (Collection of In Vitro Diagnostic, Yakuji Nippo Limited, 1997).

However, the p-nitroanilide method or carboxy-p-nitroanilide method cannot be applied to the method of measurement according to this invention because of the absorption wavelength of the reaction product. 3-carboxy-4-hydroxyanilide method, dimethylaminoanilide method or the diethylaminoanilide method, and the γ-Glu-DBHA method are such that they do not employ a reaction through which hydrogen peroxide is derived from a substance as a subject of measurement and they are applicable to the method of measurement according to this invention.

The inventors of this invention confirmed that the γ-Glu-DBHA method is suitable for the method of measurement according to this invention.

### (Determination of Blood level of Albumin)

The methods for determining the blood level of albumin in common use include, for example, the Bromocresol Green method (hereinafter referred to as BCG method) or the Bromocresol Purple method (hereinafter referred to as BCP method) (Collection of In Vitro Diagnostic, Yakuji Nippo Limited, 1997).

In any one of the methods, the determination is made by linking a pigment to albumin so as to allow the albumin to produce color. These method are such that they do not employ a reaction through which hydrogen peroxide is derived from a substance as a subject of measurement and can carry out the determination through measuring the absorption at 600 nm or more.

The inventors of this invention confirmed that the BCG method is suitable for the method of measurement according to this invention.

### (Examples of Items Measured Employing Hydrogen Peroxide Pathway)

Then the effectiveness of the method of measurement according to this invention in the reaction systems which employ the hydrogen peroxide pathway will be described taking several examples of.

First, hydrogen peroxide is produced through the reaction of a substance as a subject of measurement with an enzyme specific for the substance. Then 4-AA and a pigment source are subjected to oxidation condensation by the hydrogen peroxide via peroxidase activity. If an appropriate pigment source is selected at this point, the wavelength at which color is produced can be selected. In other words, the absorbance can be measured in the wavelength range of 600 nm or more.

In the following 8 concrete examples will be described.

### <Method for Determining the Activities of Aspartic Aminotransferase and Alanine Aminotransferase>

As methods for determining aspartic aminotransferase (hereinafter referred to as AST) and alanine aminotransferase (hereinafter referred to as ALT) in blood, the one using UV, the pyruvate oxidase (hereinafter referred to as POP)-peroxidase (hereinafter referred to as POD) method, the formazan colorimetric method, and Lightman-Frankel method are known (Collection of InVitro Diagnostic, Yakuji Nippo Limited, 1997).

The inventors of this invention confirmed that the activities of AST and ALT can be determined by applying a reagent obtained by partially modifying a commercially available measurement reagent for the POP-POD method (TA-LN Kainos: Kainos Co., Ltd.) to the method of measurement according to this invention.

### <Method for Determining Uric Acid>

As methods for determining uric acid in blood, the uricase POD method and the uricase/catalase method are known (Collection of In Vitro Diagnostic, Yakuji Nippo Limited, 1997).

The inventors of this invention confirmed that uric acid can be determined by applying a commercially available measurement reagent for the uricase-POD method (Iatro QUA, Iatron Laboratories, Inc.) to the method of measurement according to this invention.

### <Method for Determining Glucose>

As methods for determining glucose in blood, the glucose oxidase colorimetry and the UV method are known (Collection of In Vitro Diagnostic, Yakuji Nippo Limited, 1997).

The inventors of this invention confirmed that glucose can be determined by applying a commercially available measurement reagent for the glucose oxidation colorimetry (Detamina-GL-E, Kyowa Medics Co., Ltd.) to the method of measurement according to this invention.

### <Method for Determining Total Cholesterol>

As methods for determining total cholesterol in blood, the cholesterol oxidase method etc. is known (Collection of In Vitro Diagnostic, Yakuji Nippo Limited, 1997).

The inventors of this invention confirmed that total cholesterol can be determined by applying a commercially available measurement reagent for the cholesterol oxidase method (HA Test Wako, Cholesterol E - HA Test Wako, Wako Pure Chemical Industries, Ltd.) to the method of measurement according to this invention.

### <Method for Determining HDL-Cholesterol>

As methods for determining HDL-cholesterol in blood, the direct method, the magnesium dextran sulfate/magnesium phosphotungstate method are known (Collection of In Vitro Diagnostic, '99 additional print, Yakuji Nippo Limited, 1999).

The inventors of this invention confirmed that HDL-cholesterol can be determined by applying a commercially available measurement reagent for the direct method (CholesTest HDL, Daiichi Pure Chemicals Co., Ltd.) to the method of measurement according to this invention.

### <Method for Determining LDL-Cholesterol>

As a method for determining LDL-cholesterol in blood, the direct method is known (Collection of In Vitro Diagnostic, ' 99 additional print, YakujiNippo Limited, 1999).

The inventors of this invention confirmed that LDL-cholesterol can be determined by applying a commercially available measurement reagent for the direct method (LDL-EX, Denka Seiken Co., Ltd.) to the method of measurement according to this invention.

### <Method for Determining Neutral Fat>

As a method for determining neutral fat in blood, the enzymatic colorimetry is known (Collection of In Vitro Diagnostic, Yakuji Nippo Limited, 1997).

The inventors of this invention confirmed that neutral fat can be determined by applying a commercially available measurement reagent for the enzymatic colorimetry (Reagent for Automated Analyzer - HR Triglyceride - HR, Wako Pure Chemical Industries, Ltd.) to the method of measurement according to this invention.

### <Method for Determining Creatinine>

As methods for determining creatinine in blood, the Jaffe's method, the enzymatic colorimetry, the enzymatic UV method, etc. are known (Collection of In Vitro Diagnostic, Yakuji Nippo Limited, 1997).

The inventors of this invention confirmed that creatinine can be determined by applying a commercially available measurement reagent for the enzymatic colorimetry method (L Type Wako, Creatinine F, Wako Pure Chemical Industries, Ltd.) to the method of measurement according to this invention.

### (Chyle Resistance of Thermal Lens Detection Method)

An example will be described which shows that the thermal lens detection method is resistant to fine particles a few ten nm in diameter having no absorption specific for the wavelength of excitation light, for example, chyle.

In order to quantitatively determine the degree of chyle, Interference Check/A Plus (International Reagents Corporation) was used. Aserum (sample) with a formazin degree of 2100, which is generally considered to have strong chyle, was prepared using Switrol A (Nissui Pharmaceutical Co., Ltd.) in accordance with the specification. Then, after reacting HA Test Wako Cholesterol E - HA Test Wako (Wako Pure Chemical Industries, Ltd.) with the sample, measurements were made in accordance with the thermal lens detection method and the absorption spectrophotometry (635 nm).

As a result, it was confirmed that chyle caused a background when employing the absorption sepctrophotometry, but had no effect on the thermal lens detection method. This result indicates that the thermal lens detection method is resistant to chyle.

### (Excitation Light and Probe Light)

When the inventors of this invention measured hemoglobin using He-Ne laser having a wavelength of 633 nm as excitation light and Ar laser having a wavelength of 488 nm as prove light, the phenomenon was observed that the output in accordance with the thermal lens detection method was extraordinarily large and increased with time, as described in Reference Example 1 later.

This combination of excitation light and probe light, when measuring a biological item such as cholesterol after deriving a pigment such as DAOS therefrom, the output in accordance with the thermal lens detection method can be detected without any problem.

This is disclosed in, for example, International Publication WO99-64846 (International Patent Application PCT/JP99/03158 Specification "Apparatus for Analysis"; Japanese Patent Publication No. 12-2675 (Japanese Patent Application No. 10-181587 "Apparatus for Capillary Photothermal Converting Analysis"); Japanese Patent Publication No. 12-2677 (Japanese Patent Application No. 10-1617603 "Apparatus for Analysis"); Japanese Patent Application No. 10-181586 ("Apparatus and Method for Mixed Analysis"); and Japanese Patent Application No. 10-167603 ("Apparatus for Analysis").

After the inventors' intensive investigation, it was found that when irradiating methemoglobin with two types of light having respective wavelengths of 488 nm and 633 nm, an optical reaction is caused, resulting in an abnormal output value. Then, when the inventors employed laser light, as probe light, which had a wavelength longer than that of the excitation light and was not absorbed by hemoglobin, a normal output was obtained in accordance with the thermal lens detection method and hemoglobin could be quantitatively determined.

As the light source of the excitation light used in this invention to measure hemoglobin, the one is required which outputs light having a wavelength capable of exciting methehemoglobin to have an absorption band at 630 nm and sufficient to form a thermal lens. The wavelength of the excitation light is preferably 610 to 650 nm, and more preferably 630 nm ± 10 nm, based on the absorption of methehemoglobin. The types of light sources are not limited to any specific ones, as long as they have sufficient luminous intensity for the excitation at around 630 nm.

Light having a required wavelength can be taken out from, for example, a Xenon lamp using a prism, however, the output of light decreases when it passes through a prism.

Laser light having a wavelength capable of exciting a substance as a subject of measurement or light emitting diodes outputting light sufficient to excite the same can also be used. As a laser, although a He-Ne laser outputting light having a wavelength of 633 nm can be used, a semiconductor laser is suitable for the application in POC analyses and the like, since it allows a detection apparatus to be smaller. As a semiconductor laser, the one outputting light having a wavelength of 650 nm, such as TOLD 9450 MC by Toshiba, can be used; however, more preferable excitation light sources include, for example, semiconductor lasers outputting light having a wavelength of 635 nm, such as LT 050 MS by Sharp Corporation, DL-3088-011, DL-4038-025, etc. by Sanyo Electric Co., Ltd..

The wavelength of the probe light used in the measurement of hemoglobin is preferably longer than 600 nm, as described above, and more preferably longer than 630 nm ± 20 nm which is the wavelength of the excitation light. YAG lasers outputting light having a wavelength of 1064 nm and carbon dioxide lasers having about 100 oscillation lines at 900 to 1200 nm can be used; however, semiconductor lasers outputting light having a wavelength of 780 nm, such as LDT 7830 by Nippon Kagaku Engineering Co., Ltd. and DL-4034-151 by Sanyo Electric Co., Ltd. are more preferable. Semiconductor lasers outputting light having a wavelength of 830 nm, such as DL-7032-001 by Sanyo Electric Co., Ltd., LT 015 PD by Sharp Corporation and HL 8325 G by Hitachi, Ltd. can also be preferably used. In order to condense both excitation light and probe light into around a capillary, a condenser lens is required.

The excitation light sources and probe light sources described above can be applied when measuring substances other than hemoglobin.

The changes in probe light due to the thermal lens can be caught with a photodiode, a CCD camera and a photomultiplier tube. A photodiode is particularly suitable for miniaturizing a detection apparatus.

The excitation light is changed into pulse light of about 0.1 to 20 msec (frequency of 50 Hz to 10000 Hz) with a chopper etc., and the changes in quantity of the probe light synchronized with frequency of the chopper only are taken out with a lock-in amplifier. More preferably, the frequency of the chopper is in the range of 100 Hz to 4000 Hz.

The structure of the lock-in amplifier can be simplified using single-function semiconductor devices. The changing of the excitation light to pulse light may be performed by electrically modulating the semiconductor laser. In the detection of probe light, a lock-in amplifier is generally used; however, the method used in the dark field photothermal converting spectrochemical analyzer can also be used which is disclosed in Japanese Patent Publication No. 9-229883. Specifically, the method is to shield the luminous flux around optical axes of pumped light and probe light with a shield plate and detect only the probe light diverged with a thermal lens.

In detecting the changes in the thermal lens, which is formed by the excitation light changed into pulse light with a chopper, with probe light, the oscillation component (the frequency is the same as the pulse of excitation light) in the probe light only is detected. Therefore, even if the absolute amount of light I is decreased due to scattering etc., the ratio of the oscillation component i to the quantity of light I is unchanging, whereby correct values can be obtained stably.

### (Microchip)

The structure etc. of the microchip used in the method for measuring hemoglobin according to this invention is not limited to any specific one, as long as it has a capillary within which a blood sample and a measurement reagent can flow.

For example, the microchip as described below is one of the preferred forms of this invention. Specifically, the preferred microchip consists of a pair of planar members . And at least one of the pair of planar members includes a groove on the surface of which liquid can flow. The two planar members are bonded together with the surface including the groove facing inside so as to form a capillary inside the microchip.

Such a microchip can be produced using inorganic materials, such as silicon and glass, or organic polymers. However, the detection portion in the thermal lens detection method must be transparent to both excitation light and probe light.

In the production of a microchip using silicon or glass as the material, an etching protective film (Cr etc.) is formed on a substrate of glass, quart or Si to a few hundred nm in thickness by the vacuum evaporation coating etc. Then substrate with the film is coated with a patterning resist with a spinner etc. The resist is exposed to ultraviolet light using a photolithographic mask, and then developed (remove uncured portion with a solvent), followed by patterning to give a desired pattern.

Patterning is performed again using the patterned resist as an etching mask by dissolving and removing the etching protective film with an aqueous solution of potassium ferricyanide etc. Then the substrate is etched with, for example, an aqueous solution of hydrofluoric acid while using the patterned resist and the etching protective film as a mask, to form a groove. After this, the resist and the protective film are etched and removed. In addition to the above substrate, a substrate of, for example, glass is prepared which has through holes formed therein by the laser processing or ultrasonic processing.

Finally, the substrate provided with a groove and the substrate provided with through holes are put together with the surface including a groove facing inside, heated, for example, in a vacuum furnace (when both the substrates are glass ones, they are heated at about 600°C for a few hours), and cooled naturally so as to allow them to fuse. Thus a microchip is produced.

When producing a microchip using organic polymer, resin must be used which is transparent to the light used for measurements, since the microchip is used for optical detection. In the measurements in accordance with the photothermal converting detection method, resin can be suitably used of which light transmittance measured with ASTM D1003 is 80% or more in the wavelength rage of 600 nm to 800 nm, and more preferable 90% or more. The above light transmittance shows the value obtained by subtracting the sum of the reflectance on the surface of the microchip and the absorptance of the organic polymer substrate itself from 100%.

The light scattered on the surface of the microchip has no effect on the organic polymer, but on the other hand, the light absorbed by the organic polymer substrate has an effect on the organic polymer, allowing it to produce heat. Therefore, when light passes through the organic polymer, an effect just like a thermal lens is produced and it becomes a background of the output in accordance with the thermal lens detection method, which may cause error in measurements. Accordingly, estimate must be made of the organic polymer to be used before producing a microchip, and the range of transmittance must be investigated in which light has no adverse effect on the thermal lens detection method.

When measurements are made in accordance with the absorption spectrophotometry, even if the organic polymer absorbs about 10% of incident light, the quantity of light is only decreased to 90%, which does not affect the detection sensitivity significantly. When measurements are made in accordance with the photothermal converting detection method, however, even if the organic polymer absorbs 10% or less of incident light, the results of measurement is affected significantly, due to the thermal lens formed in the microchip of organic polymer. In particular, when high sensitivity is required for measurements, that is, when the concentration of the substance as a subject of measurement is low and the capillary is narrow (the groove is shallow), even if the organic polymer absorbs only 1% or less of incident light, and in some cases, even if the organic polymer absorbs as very small as 0.5% or less of incident light, the results of measurement is likely to be affected adversely.

For example, suppose that measurements are made for a substance (liquid), which has an absorbance of about 0.1 in a cuvet 1 cm in size, using a microchip of organic polymer including a capillary 50 µm in size (that is, optical path length is 50 µm). The absorbance 0.1 at an optical path length of 1 cm then corresponds to the absorption rate 0.103% in a capirally of 50 µm in size. If the allowable absorption by the organic polymer portion of the organic polymer microchip (formation of a thermal lens) is up to 10 times of the above value, the absorption rate becomes 1%, and if the same is up to 2 times of the above value, the absorption rate becomes 0.2%.

In other words, in order to measure a substance having an absorbance of about 0.1 at an optical path length of 1 cm is carried out in accordance with the method of this invention, the absorption of light by the organic polymer constituting a microchip is 1% or less and more preferably 0.2% or less.

However, the above values can be changed depending on the concentration of a substance as a subject of measurement and the narrowness of a capillary. For example, if the absorbance in a cuvet 1 cm in size is increased to about 0.5 by increasing the concentration of the substance as a subject of measurement, the absorbance corresponds to the absorption rate of 0.342% in a capillary of 50 µm in size. Accordingly, if the allowable absorption by the organic polymer portion of the organic polymer microchip (formation of a thermal lens) is up to 10 times of the above value, the absorption rate of the organic polymer microchip becomes 3.5%, and if the same is up to 2 times of the above value, the absorption rate becomes a little less than 1%.

The workability when providing the aforementioned planar member with a groove is also an important factor in the selection of resin type. The types of resin satisfactorily used in terms of its workability include, for example, the ordinary thermoplastic resin which can undergo melt processing and the resin obtained by UV curing. And the one particularly satisfactorily used is thermoplastic resin which can undergo melt processing, because planar members having a groove on their surface can be produced abundantly and inexpensively. Among the thermoplastic resin, amorphous thermoplastic resin, thermoplastic polymer alloy containing amorphous resin as a main ingredient, or some crystalline thermoplastic resin with a low crystallinity are suitable.

Affinity for blood, in particular, causing no aggregation, solidification and activation of platet is one of the requirements of the preferable materials.

The types of resin which satisfy the light transmitting characteristic and the workability described above and can be particularly satisfactorily used include, for example, styrene resin such as polystyrene, styrene-acrylonitrile copolymer; methacryl resin such as polymethylmethacrylate (PMMA) and methylmethacrylate-styrene copolymer; polycarbonates (PC); polysulfones; polyetersulfones; polyeterimides; polyarylates; and polymethylpentene.

1,3-cyclohexadiene polymers are also suitably used. They can be used in the form of a homopolymer; however, when they are used in the form of a copolymer, they can form copolymers with the compounds, for example, chain conjugate diene monomers such as 1,3-butadiene, isoprene, 1,3-pentadiene and 1,3-hexadiene; vinyl aromatic monomers such as styrene, α-methylstyrene, p-methylstyrene, 1,3-dimethylstyrene, vinylnaphthalene and vinylstyrene; polar vinylmonomers such as methyl methacrylate, methyl acrylate, acrylonitrile, methyl vinyl ketone and methyl α-cyanoacrylate; polar monomers such as ethyleneoxides, propylene oxide, cyclic lactone, cyclic lactam and cyclic siloxane; or ethylene, α-olefin monomers. The copolymerization ratio of 1,3-cyclohexadiene monomer/comonomer in this case is preferably 75/25 to 100/0.

Cyclohexadiene polymers having a high light transmittance are described in detail in Japanese Patent Application No. 9-277045 and Japanese Patent Application No. 10-287007. These polymers have little absorption in the wavelength range of 200 nm.

The aforementioned organic polymer planar member having a groove therein can be formed by the methods, such as machinery cut of a planar plate, etching processing using laser etc., UV cure or thermal cure of monomer or macromonomer in a mold, and melt processing or plastic processing of thermoplastic resin. The molding processing satisfactorily employed is melt processing or plastic processing of thermoplastic resin, because the aforementioned planar members can be produced abundantly and inexpensively. The molding processing much more satisfactorily employed is injection molding, compression molding or embossing molding of thermoplastic resin using a metal mold.

In particular, a type of injection molding which carries out molding processing while lowering the solidification temperature of the resin surface in contact with a metal mold during the process of filling the mold cavity with resin (disclosed in Japanese Patent Publication No. 10-128783 and Japanese Patent Application No. 10-46665) can form planar members having a fine groove with high molding accuracy in high productivity. One of the concrete examples of this type of injection molding is carried out while filling the cavity of the mold with carbon dioxide. The pressure of the carbon dioxide in this case is preferably 10 MPa or less and more preferably 0.3 to 2 MPa.

Another type of injection molding in which molding is carried out while heating the surface of the mold, for example, by the high frequency induction heating right before the molding (described in Japanese Patent Publication No. 62-58287, U.S. Patent No. 4439492) and by the radiant heating right before the molding (described in Seikei Kako Symposia, '95, 241 (1995), Seikei Kako, '96, 69 (1996), Synthetic Resin, vol.42(1), 48 (1992), etc.) are also preferable when producing a microchip for use in this invention.

The reason is that the above type of molding method allows both mold surface transfer characteristics and molding cycle to be compatible with each other by setting the temperature of a mold low and selectively heating the mold surface alone right before the molding by the high frequency induction heating or with a heat source such as halogen lamp.

As a metal mold for molding the microchip as above, the ones which are generally used for molding synthetic resin and consist of metal, such as iron or steel containing iron as a main ingredient, aluminum, alloys containing aluminum as a main ingredient, zinc alloys and beryllium-copper alloys can be satisfactorily used.

One example of the methods for producing a metal mold will be shown below. First a matrix having a surface configuration of an organic polymer planar member including an intended fine groove is prepared from a material such as metal, plastic, silicon or glass by the method, such as machinery cut, etching processing, or photolithographic processing of ultraviolet cured resin. Then a metal mold is produced from this matrix by the electrochemical casting method.

The metal mold can also be produced using the method, which is disclosed in Japanese Patent Publication No. 6-283830, for forming a resist pattern. After forming a resist pattern on a metal substrate, the portion with no resist coated thereon is coated with metal plating. Then the resist is removed and a metal plate provided with a fine pattern is formed on the substrate surface. Processing of resin and sintered glass and the like can be performed using this metal plate as a metal mold.

The organic polymer microchip for use in this invention can also be produced based on the method for producing a circuit substrate disclosed in Japanese Patent Publication No. 6-283830.

The methods for producing a microchip using a glass substrate include, for example, the one in which a microchip is produced by forming a resist pattern on a glass substrate and processing the glass substrate by the sand blast method. According to this method, all the particles coming flying are directed uniformly vertically due to the thick resist, whereby the glass substrate can be processed sharply compared with the ordinary thin resist and a groove having a high aspect ratio can be formed.

Further, a method can also be employed in which a microchip is produced in the following steps of: coating a substrate of glass or resin with photosensitive resist, exposing the portion other than the groove; and removing the uncured portion to form a resist pattern in the form of a groove.

Further, a microchip applicable to this invention can also be produced in the following steps of: providing a glass substrate with a groove running through from one side to the other using laser etc.; and sandwiching the substrate with 2 sheets of glass planar plates.

Further, a method can also be employed in which a microchip is produced in the following steps of: forming a resist pattern on a glass substrate; and wet etching the glass substrate with hydrogen fluoride etc. to form a groove thereon.

In the aforementioned microchip which consists of planar members including a groove, the inner surface of the groove may undergo protein adsorption preventive treatment with polyethylene glycol etc. Further, when using electroendosmosis flow, which will be described later, as a liquid supplying means, the inner surface of the groove may be surface treated so as to create a stable electroendosmosis flow.

The microchip used in this invention preferably consists of two sheets of planar members . At least one of the two members includes a groove as described above and the two members are put together with the surface including a groove facing inside . Then, they undergo ultrasonic melt processing, hot melt processing, adhesion with adhesives such as hot melt adhesive and UV adhesive, sticking with sticking agents, or pressing directly or via a thin elastic sheet to constitute an integrally formed microchip.

The material for the planar member including no groove (hereinafter referred to as covering planar plate) can be selected from the materials used for the planar member including a groove. Moreover, the thin glass planar plates and the like can be used. The materials for the two planar members may be the same or different. The thickness of the planar plates is not limited as long as it is not an obstacle to measurements; however, it is preferably in the range of about 0.05 to a few mm. Whatever material is selected, the important point is that the absorption rate at the detecting portion in accordance with the thermal lens detection method is 5% or less and more preferably 1% or less, as described above.

Preferably, the microchip used in this invention is constructed with two sheets of planar plate members as described above from the view point of productivity. Preferably, at least one of the two members includes a groove and the microchip is constructed in such a manner as to bond the two members together with the surface including a groove facing inside. However, the microchip can be constructed with three sheets of planar plate members in such a manner as to sandwich one planar plate member with a groove pierced therein with two other planar plate members.

The above covering planar plate may be provided with a through hole as a reservoir or may be mounted with a cylindrical reservoir (including a waste liquid trap) in such a manner as to allow it to project therefrom. The size of the reservoir is not limited, however it is preferably 1 to a few mm in height and 1 to a few mm in diameter. When both the planar plate including a groove and the covering planar plate are about a few mm thick, the above through hole can also serve as a waste liquid trap.

The flow rate used in this invention means the volume of the liquid moving within the groove (capillary) within a fixed period of time.

The profile of the groove provided on the surface of the above planar plate member through which a liquid flows is preferably rectangular; however, the groove may have other polygonal shapes such as triangle, semi-circle, semi-ellipsoid and others . The microchip may have a flow path consisting of several types of different shaped grooves in combination. However, with respect to the detecting portion in accordance with the thermal lens detection method, the profile of the groove is preferably rectangular. The width of the upper side (open side) of the groove may be the same as that of the lower side (bottom) or wider than that of the lower side.

If the groove is too small, clogging may be caused due to the fine particles or blood cells mixed in the sample for measurement. If the groove is too large, the efficiency of mixing two types of liquid is decreased when they join together. Accordingly, the portion through which blood passes is required to be 20 to 500 µm in width and 20 to 1000 µm in depth. Preferably it is 50 to 300 µm in width and 30 to 200 µm in depth.

When hemolyzing blood cells in the measurement of hemoglobin etc., the flow path after hemolyzing operation is preferably 1 to 1000 µm in width, 0.1 to 1000 µm in depth and 1 to 1,000,000 µm² in cross-sectional area. More preferably it is 30 to 1000 µm in width, 20 to 1000 µm in depth and 600 to 1, 000, 000 µm² in cross-sectional area and the width is the same as the depth or larger than the same.

Preferably, the dimensional accuracy of the groove in the above planar plate member is high from the view point of carrying out analyses and measurements for trace components. Specifically, the dimensional accuracy (dimension trans ferring accuracy) of the groove is preferably within a tolerance of ±5% in width and depth and within a tolerance of ±7% in cross-sectional area so as to obtain accuracy of operation and reproducibility among analyzers. In order to make measurements with a high accuracy, preferably the dimensional accuracy of the groove is preferably within a tolerance of ±2% in width and depth and within a tolerance of ±4% in cross-sectional area.

### (Method for Supplying Liquid)

In this invention, a quantitative reaction within a microchip can be carried out in such a manner as to measure the blood sample and the measurement reagent to be used in a fixed amount with a measuring tank and mix them inside or outside of the microchip. Or a quantitative reaction within a microchip can be carried out continuously over at least a certain period of time in such a manner as to mix the measurement reagent and the sample in a fixed ratio by controlling the flow rate ratio of the blood sample to the measurement reagent and allow them to react with each other (Japanese Patent Application No. 10-181586 described above, "Apparatus and Method for Mixed Analysis").

As a liquid supplying method, the one can be used which utilizes a pump outside the chip, centrifugal force, or gravity as liquid supplying means.

Further, liquid can be supplied utilizing electrophoresis or electroendosmosis flow generate by applying electric field to the liquid in the groove (capillary) (described in detail "Capillary Electrophoresis" Kodansya Publishing Company).

Electroendosmosis flow is such that liquid in a capillary moves with the movement of ions on the inner surface of the capillary. When the capillary is formed of glass or silicon, protons of silicic acid on the glass surface serve as a driving force. Even when the microchip is formed of organic polymer such as PMMA and PC and there exists no special ion species on the inner surface of the capillary, electroendosmosis flow can be generated, depending on the composition of the liquid flowing through the capillary, in such a manner as to allow the inner surface of the capillary to adsorb electrolyte in the liquid and utilize the movement of the electrolyte. In order to generate a stable electroendosmosis flow, organic polymer having sulfonic acid groups or carboxylic acid groups may be added to the inner surface of the capillary through graft polymerization. Further, the method is also effective to form carboxylic acid groups on the inner surface of the capillary by hydrolyzing the surface of PMMA etc. with an aqueous solution of sodium hydroxide etc.

In the method utilizing electroendosmosis flow, the accurate flow rate can be controlled in a delicate and adaptable manner by controlling voltage, or in accordance with the program set in advance. Accordingly the reaction or isolation in a microchip can be controlled with a high accuracy. In this respect, the method is preferred.

As a source for generating electroendosmosis flow, a high voltage generator (for example, Model HCZE-30PNO, 25 by Matsutei Precision Inc., voltage up to 30 kV is applicable) is used. The output of the high voltage generator can be controlled with an external computer via an interface board (for example, DAQCard-1200, CB-50 connector block, by National Instruments Co., Ltd.). The program for timing the application of voltage can be prepared using, for example, NI-DAQ drive soft ware (LabVIEW).

Liquid can be supplied using gravity as a driving force, and as to this liquid supplying method, the inventors of this invention have already filed a patent application (Japanese Patent Application No. 11-352445).

### (Form of Capillary (Channel) in Microchip)

The flow path (capillary) provided in a microchip so as to mix or dilute a measurement reagent and a blood sample can take the form in which a single channel is joined with another channel, or in which a single channel is joined with more than one different channel at one point. Joining a single channel with another channel or more than one different channel to form a single channel makes it possible to carry out mixing operation and diluting operation. Further, changing the flow rate at these operation makes possible mixing and dilution at different ratios.

The mixing or diluting ratio can be determined by mechanically changing the flow rate of each of the channel joined together, when the liquid supplying means is a pump. When the liquid supplying means is electroendosmosis flow, the flow rate of each of the channels joined together can be regulated by changing the cross-sectional area or length of each channel, changing the way to apply voltage thereto, or changing the charged state of the inner surface of capillary thereof by the surface treatment and the like.

When the liquid supplying means is centrifugal force, each flow path must be designed taking into consideration the distance from the center, the number of revolution, etc.

The liquid can be treated continuously from mixing operation to reaction, and to measurement in a continuous flow path, without taking the trouble of picking up and measuring a fixed amount of the liquid for separation after reacting the blood sample and the measurement reagent with each other.

In the method in which the mixing ratio can be specified by the flow rate, reaction need not be carried out for a long period of time continuously. For example, if the mixing operation takes 10 seconds, at least 10 seconds (usually about 20 seconds, a little too much) are spent joining of a blood sample and a measurement reagent, the mixture is allowed to move through the channel for such a period of time to be sufficient for their reaction, and again at least 10 seconds are spent joining of the mixture and another measurement reagent. Measurement is made after allowing the mixture to move through the channel for such period of a time to be sufficient for their reaction.

A mixture of a blood sample and a measurement reagent can also be supplied to a thermal lens detection apparatus in such a manner as to provide the microchip with a measuring tank (graduated cylinder), measure the blood sample and the measurement reagent with the tank, and mix them.

In this invention, preferably 2 to 10 minutes are spent continuing the reaction of a sample blood and a measurement reagent so as to mix them and to complete their reaction.

### (Experimental Example 1: Examination of Mixing Ratio of Blood Sample and Measurement Reagent and Concentration of Oxidant)

The effect of the mixing ratio of a measurement reagent to a blood sample on the absorbance relative to excitation light at a wavelength of 633 nm, a requirement of the thermal lens detection method, was examined. As a measurement reagent (hemolyzing oxidation reagent liquid), was used a solution (pH 6.0) in which potassium ferricyanide (K₃Fe(CN)₆), 2-(N-morpholino) ethanesulfonic acid (hereinafter referred to as MES) and Triton X-100 were dissolved 0.61 mM, 10 mM and 0.8% by weight in concentration, respectively.

As a blood sample, heparinized blood collected from a healthy subject was used. When the mixing ratio of the blood sample to the above measurement reagent was 1: 250, 996 µl of the above measurement reagent was added to 4 µl of the blood sample; when the mixing ratio is 1: 415, 996 µl of the above measurement reagent was added to 4 µl of the blood sample diluted with PBS (phosphoric acid buffer solution) to 60%; and when the mixing ratio is 1: 1245, 996 µl of the above measurement reagent was added to 4 µl of the blood sample diluted with PBS to 20%.

The changes in the absorbance with time since the instance of reagent addition are shown in Figure 1 (mixing ratio 1 : 250), Figure 2 (mixing ratio 1 : 415) and Figure 3 (mixing ratio 1 : 1245). After 1200 seconds (20 minutes) elapsed since the instance of reagent addition, the absorbance was decreased by 8.5% in the mixing ratio of 1 : 250, 16.3% in the mixing ratio of 1 : 415, and 31% in the mixing ratio of 1 : 1245.

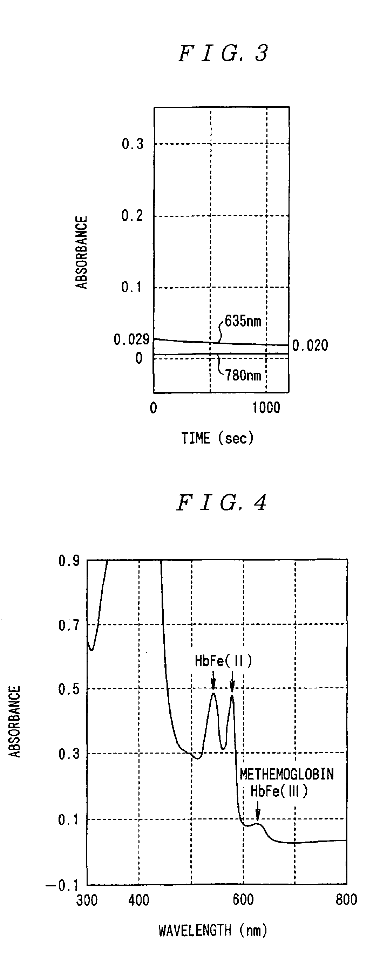

When the mixing ratio was 1 : 4, even though 800 µl of the above reagent and 200 µl of the blood sample were mixed and left to stand at room temperature for 5 minutes, the oxidation of hemoglobin to methemoglobin was insufficient, as shown in Figure 4.

Then, the concentration of the oxidant in the measurement reagent was increased to give a measurement reagent in which potassium ferricyanide, MES and Triton X-100 were dissolved 10 mM, 12.5 mM and 1% by weight in concentration, respectively (pH 6.0).

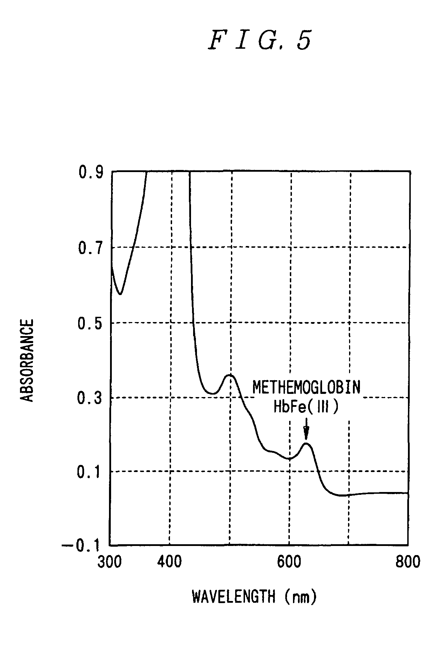

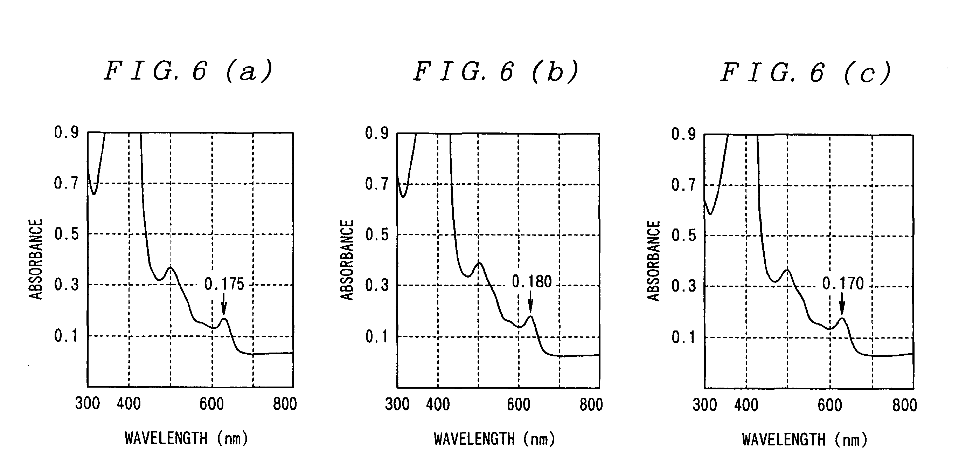

200 µl of the blood sample and 800 µl of the measurement reagent, of which oxidant content was increased, were mixed with each other, and the absorbance was measured after 5 minutes. As a result, the oxidation of hemoglobin was allowed to progress, as shown in Figure 5. The absorption spectra under these conditions 30 seconds, 10 minutes, 20 minutes after mixing were shown in Figure 6. In this case, after 1200 seconds (20 minutes) elapsed since the instance of reagent addition, the absorbance was decreased by 2.9%, indicating stable absorbance.

### (Experimental Example 2: Examination of the Effect of pH Value)

pH value was examined using as a measurement reagent a solution in which potassium ferricyanide and Triton X-100 were dissolved 0.61 mM and 0.8% by weight in concentration, respectively. The pH value was adjusted using reagents for pH adjustment shown below. For pH values of 2 and 4, a reagent prepared by adjusting 10 mM citric acid buffer with NaOH was used; for pH value of 6, a reagent of 10 mM MES buffer; for pH value of 8, a reagent of 10 mM of TRIZMA (Sigma Co., Ltd.); for pH value of 10, a reagent of about 1 mM of NaOH solution; and for pH value of 12, a reagent of about 10 mM of NaOH solution.

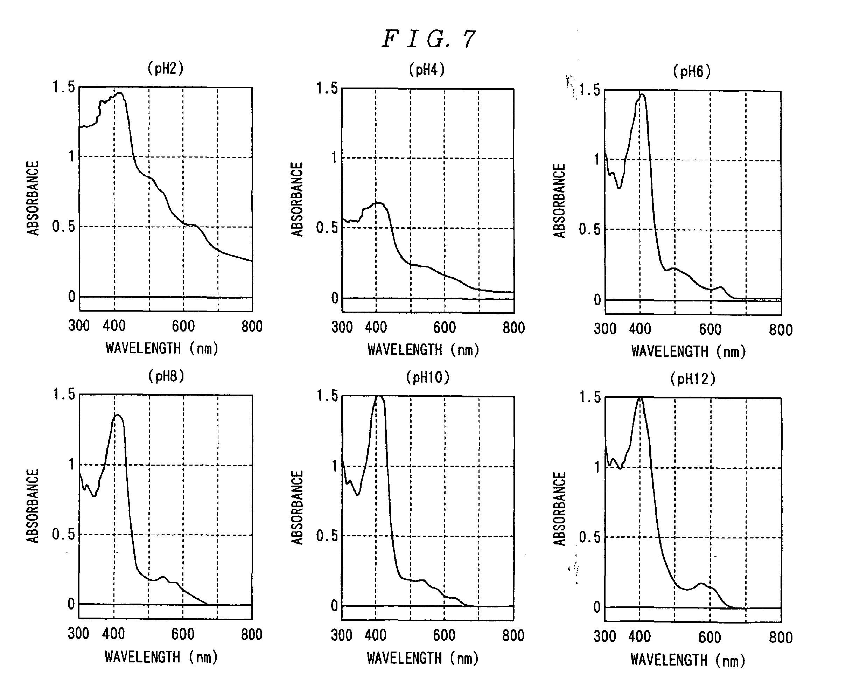

The absorbance at 633 nm is shown in Figure 7. Methemoglobin has an absorption peak at 630 nm when pH values are 2, 6 and 10, however it has a shoulder, but no absorption peak when pH values are 4, 8 and 12. The background absorbance at 700 nm was 0.34 at pH 2, 0.05 at pH 4, 0.02 at pH 6, 0.00 at pH 8, 0.00 at pH 10, and 0.00 at pH 12.

Consequently, according to the method for measuring substances of this invention, concentration of substances such as hemoglobin can be measured from a very small amount of sample, in particular, a very small amount of sample obtained from living organism, such as blood or urea. Further, the method of this invention allows using of laser light having a long wavelength as excitation light, whereby a thermal lens detection apparatus can be produced inexpensively, that is, the use of the method is economical. Thus, the method for measuring substances of this invention and the measurement reagent used in the method are suitable for POC analyses and the like.

Further, the method for measuring hemoglobin of this invention employs a measurement reagent which contains no cyanide such as potassium cyanide, which is a poisonous substance; therefore, troublesome waste liquid disposal becomes unnecessary.

### Brief Description of the Drawings

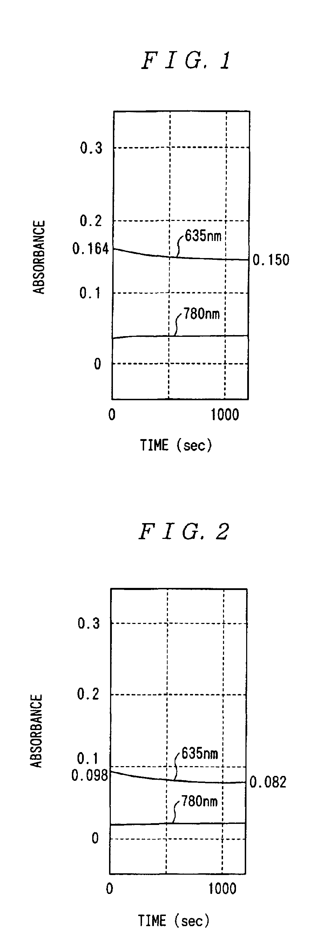

Figure 1 is a graph showing the change with time in absorbance at 635 nm (upper curve) and 780 nm (lower curve) of the sample for measurement which is prepared by mixing a blood sample and a measurement reagent in the mixing ratio of 1 : 250 in the experimental example 1;
Figure 2 is a graph showing the change with time in absorbance at 635 nm (upper curve) and 780 nm (lower curve) of the sample for measurement which is prepared by mixing a blood sample and a measurement reagent in the mixing ratio of 1 : 415 in the experimental example 1;
Figure 3 is a graph showing the change with time in absorbance at 635 nm (upper curve) and 780 nm (lower curve) of the sample for measurement which is prepared by mixing a blood sample and a measurement reagent in the mixing ratio of 1 : 1245 in the experimental example 1;
Figure 4 is a graph of the absorption spectrum of the sample for measurement which is prepared by mixing a blood sample and a measurement reagent in the mixing ratio of 1 : 4 and allowed to react for 5 minutes in the experimental example 1 (the concentration of the oxidant in the measurement reagent is the same as those shown in Figures 1 to 3);
Figure 5 is a graph of the absorption spectrum of the sample for measurement which is prepared by mixing a blood sample and a measurement reagent in the mixing ratio of 1 : 4 and allowed to react for 5 minutes in the experimental example 1 (the concentration of the oxidant in the measurement reagent is higher than that shown in Figure 4);
Figure 6 is a graph showing the change with time in the absorption spectrum of the sample for measurement which is prepared by mixing a blood sample and a measurement reagent in the mixing ratio of 1 : 4 in the experimental example 1 (the concentration of the oxidant in the measurement reagent is the same as that shown in Figure 5), Figure (a) showing the change after 30 seconds, Figure (b) after 10 minutes, and Figure (c) after 20 minutes;
Figure 7 is a graph of the absorption spectrum of the sample for measurement measured under the condition of pH 2, 4, 6, 8, 10 and 12 in the experimental example 2;

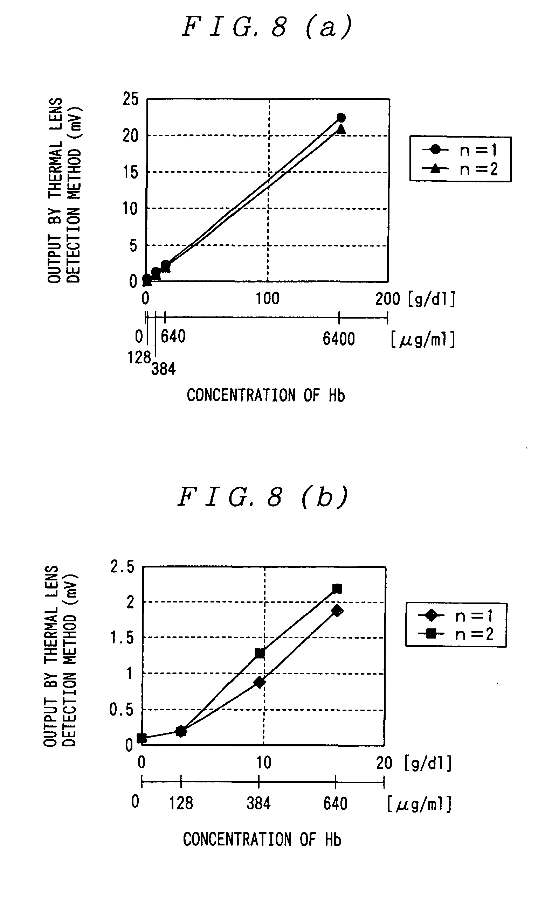

Figure 8 (a) is a graph showing the relationship between the hemoglobin concentration and the output in accordance with the thermal lens detection method obtained from the experimental example 3, Figure 8(b) is an enlarged view of the portion shown in abscissa of Figure 8 (a) in which the concentration of hemoglobin is low, the numbers with a unit g/dl in the upper row in abscissa showing the hemoglobin concentration in a blood sample, the numbers with a unit µg/ml in the lower row in abscissa showing the hemoglobin concentration in a sample for measurement.

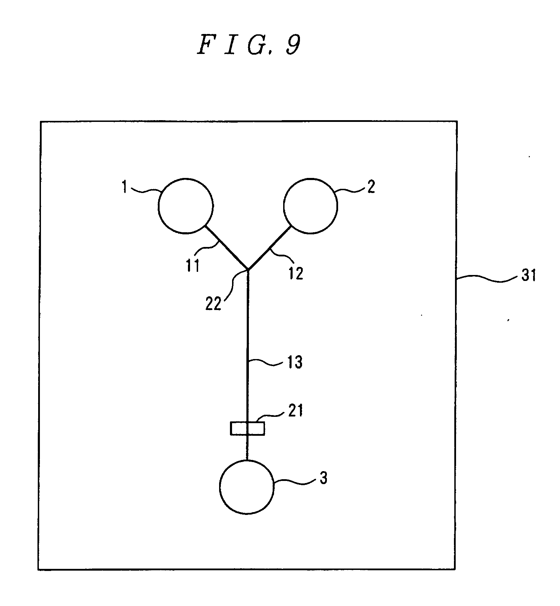

Figure 9 is a view showing the geometry of a flow path in an organic polymer microchip;
Figure 10 is a graph showing the results of ALP activity measurements made in accordance with the photothermal converting detection method in a glass cuvet;
Figure 11 is an ALP activity chart showing the results of ALP activity measurements made in accordance with the photothermal converting detection method in the comparative example 1;
Figure 12 is a graph showing the results of ALP activity measurements made in accordance with the absorption spectrophotometry,

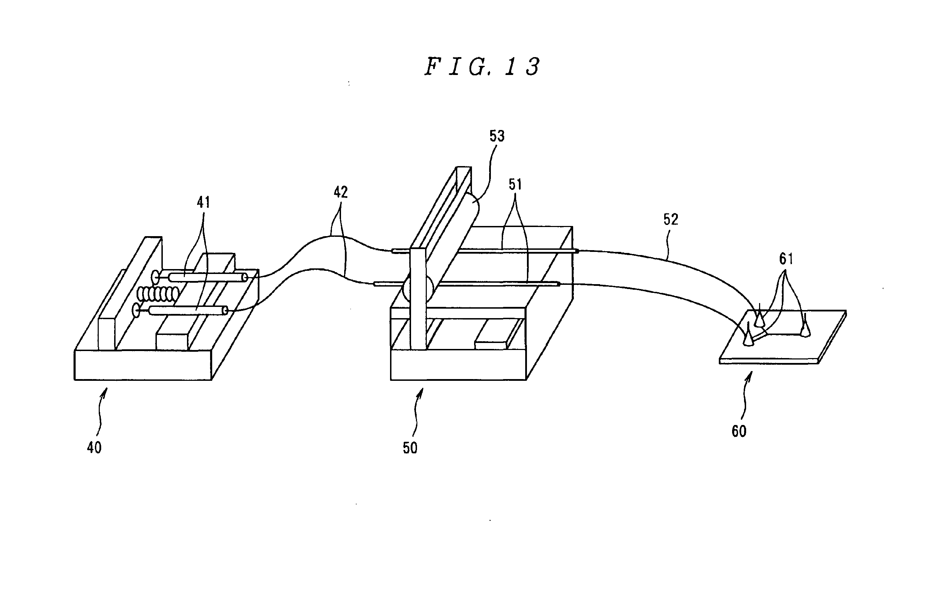

Figure 13 is a schematic view showing the structure of the liquid supplying system in the example 5:

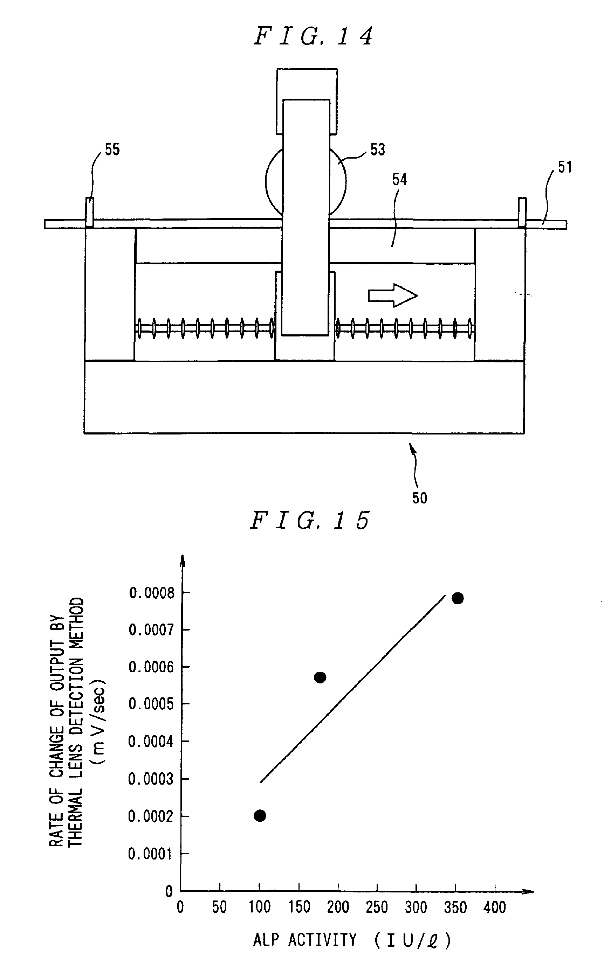

Figure 14 is a schematic view illustrating the structure of the Perista pump of Figure 13;
Figure 15 is a graph showing the results of ALP activity measurements made in accordance with the photothermal converting detection method in a resin microchip;
Figure 16 is a graph showing the results of measurements for BCI2 particle size distribution;

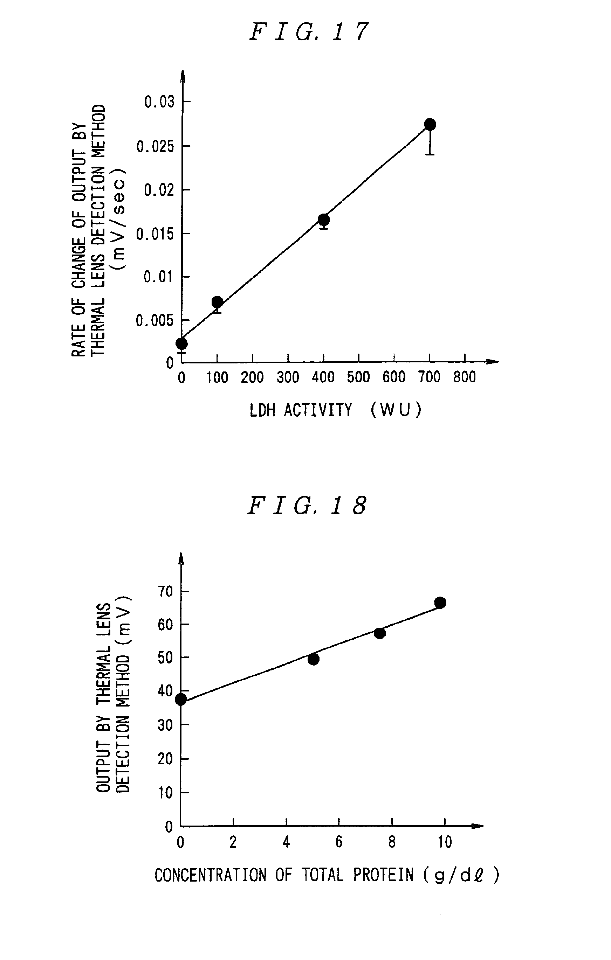

Figure 17 is a graph showing the results of LDH activity measurements made in accordance with the photothermal converting detection method;
Figure 18 is a graph showing the results of total protein concentration measurements made in accordance with the photothermal converting detection method;

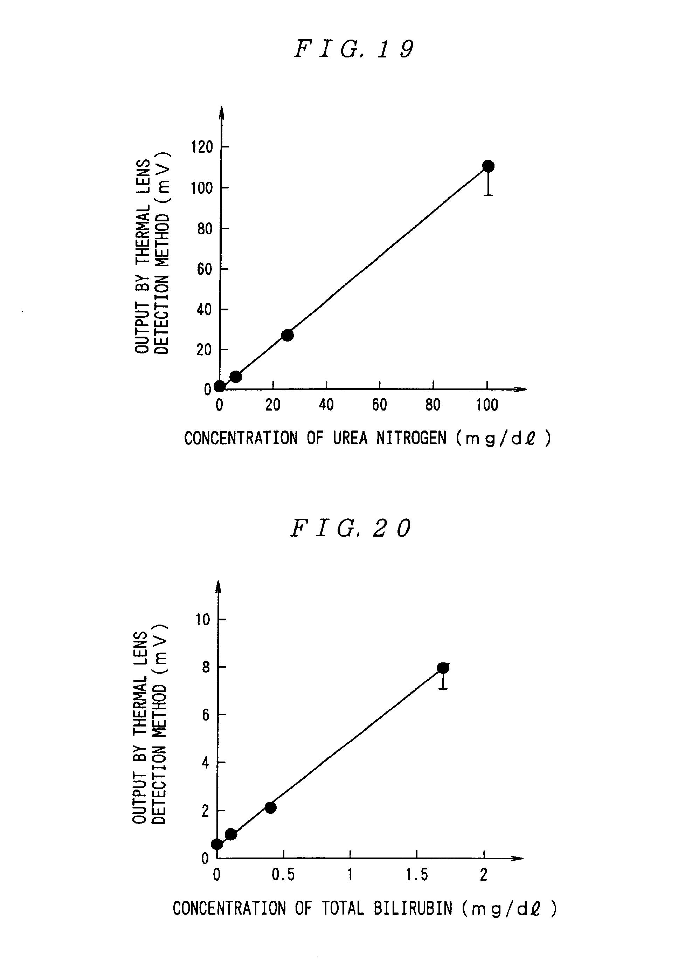

Figure 19 is a graph showing the results of urea nitrogen concentration measurements made in accordance with the photothermal converting detection method;
Figure 20 is a graph showing the results of total bilirubin concentration measurements made in accordance with the photothermal converting detection method;

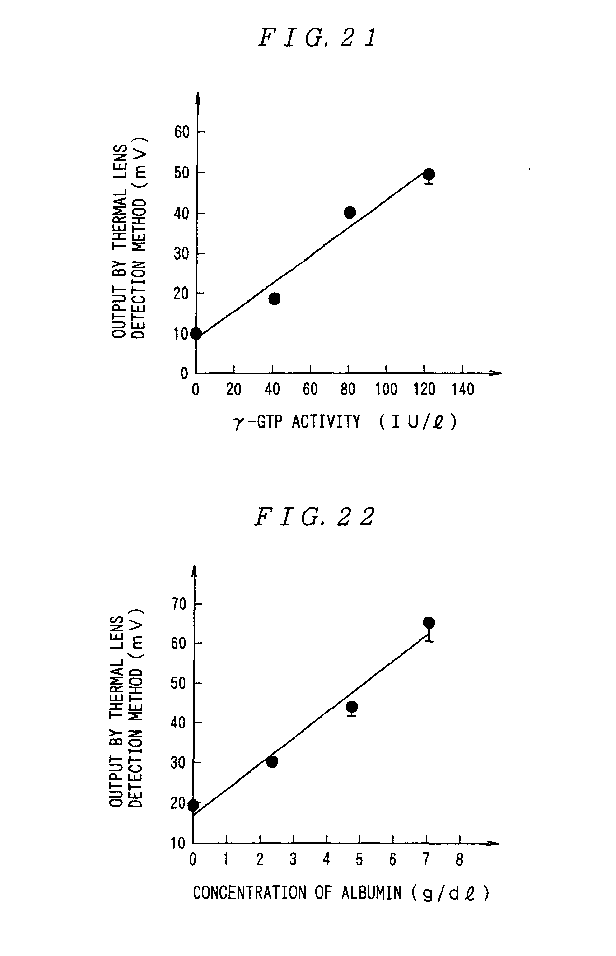

Figure 21 is a graph showing the results of γ-GTP activity measurements made in accordance with the photothermal converting detection method;
Figure 22 is a graph showing the results of albumin concentration made in accordance with the photothermal converting detection method;

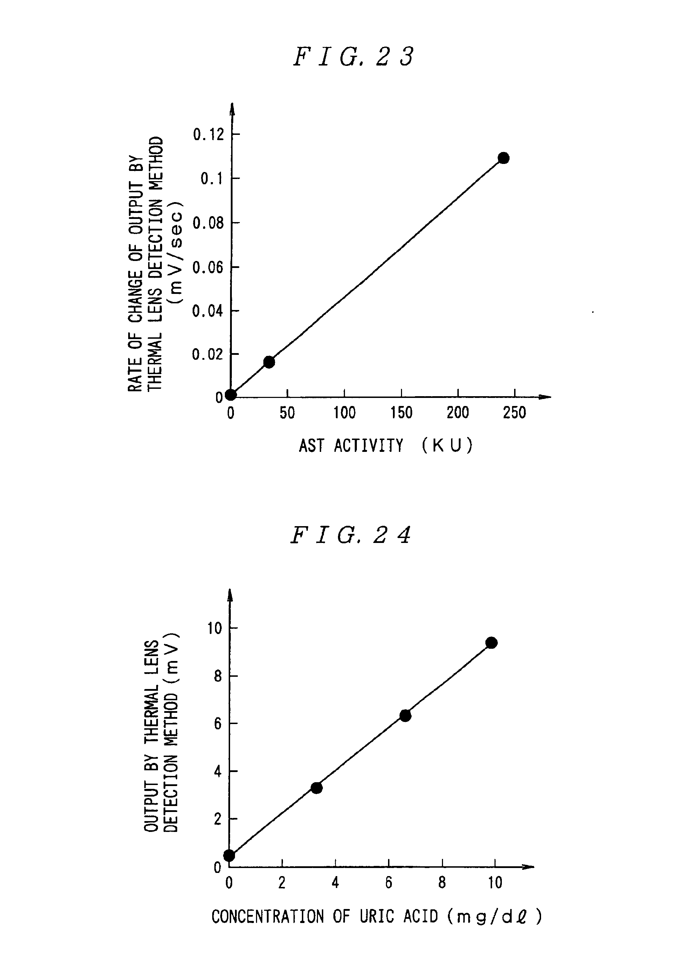

Figure 23 is a graph showing the results of AST activity measurements made in accordance with the photothermal converting detection method;
Figure 24 is a graph showing the results of uric acid concentration measurements made in accordance with the photothermal converting detection method;

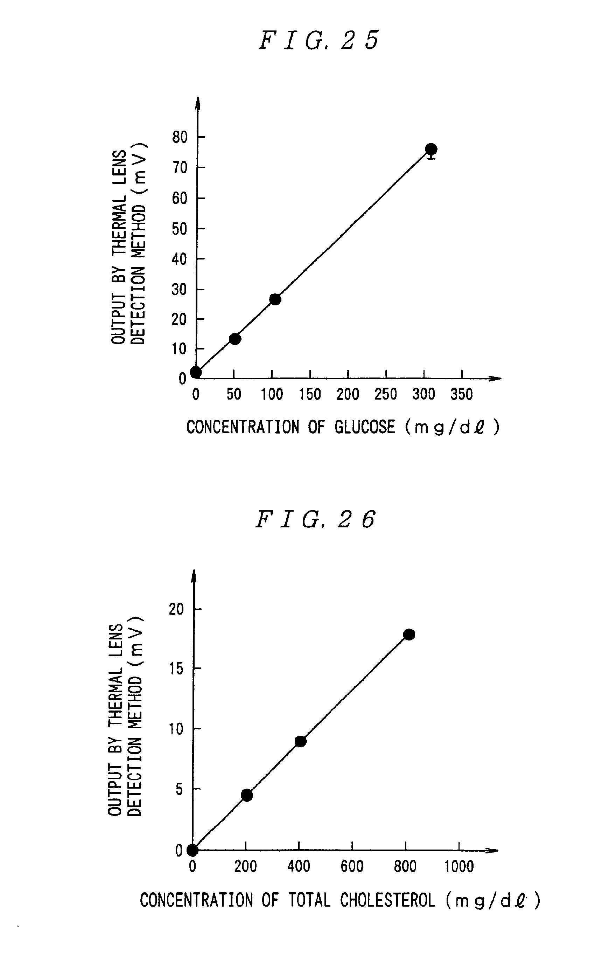

Figure 25 is a graph showing the results of glucose concentration measurements made in accordance with the photothermal converting detection method;
Figure 26 is a graph showing the results of total cholesterol concentration measurements made in accordance with the photothermal converting detection method;

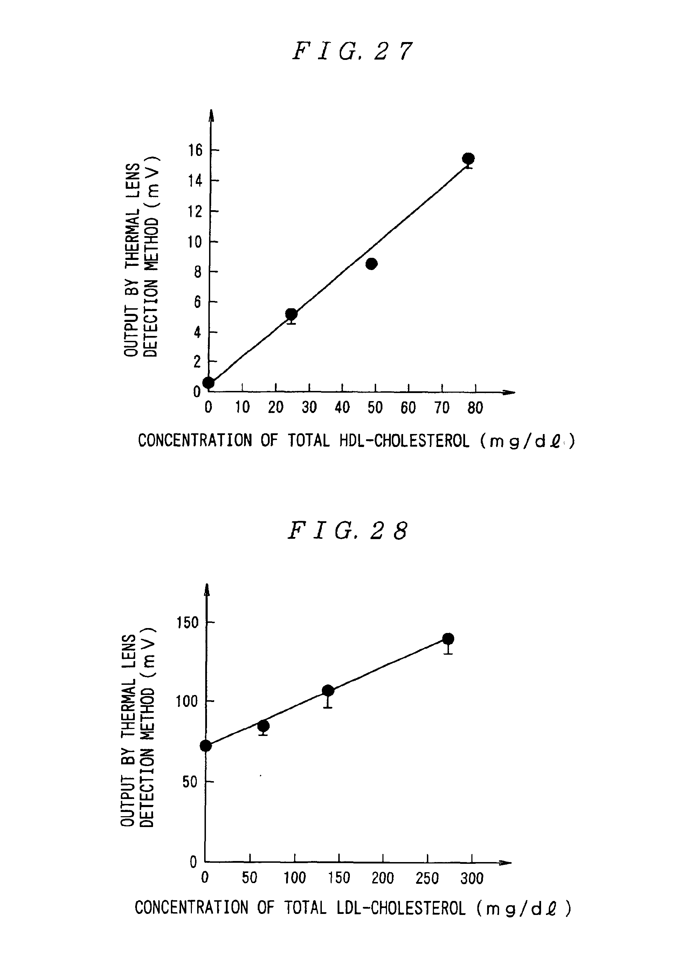

Figure 27 is a graph showing the results of HDL-cholesterol concentration measurements made in accordance with the photothermal converting detection method;
Figure 28 is a graph showing the results of LDL-cholesterol concentration measurements made in accordance with the photothermal converting detection method;

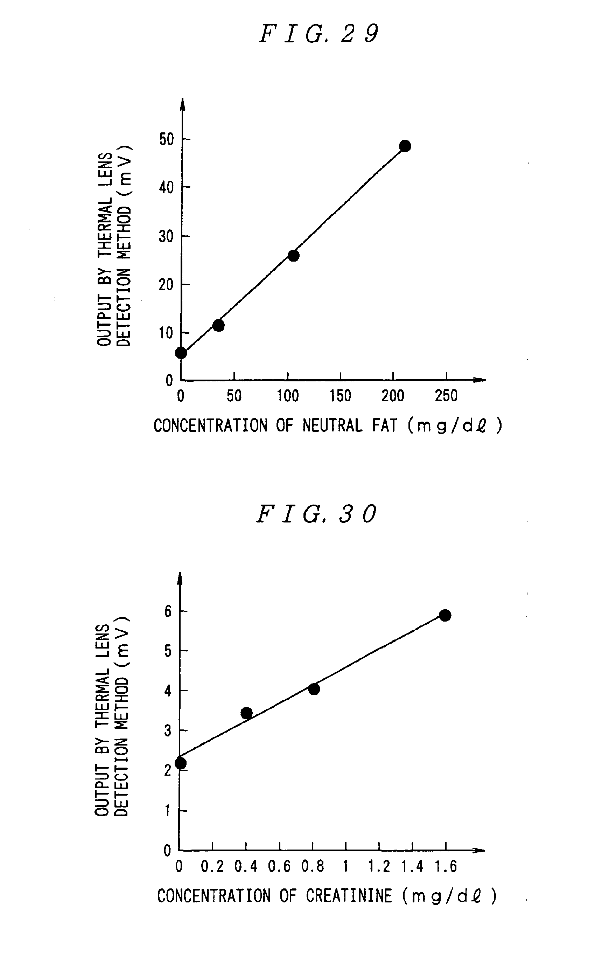

Figure 29 is a graph showing the results of neutral fat concentration measurements made in accordance with the photothermal converting detection method;
Figure 30 is a graph showing the results of creatinine concentration measurements made in accordance with the photothermal converting detection method;

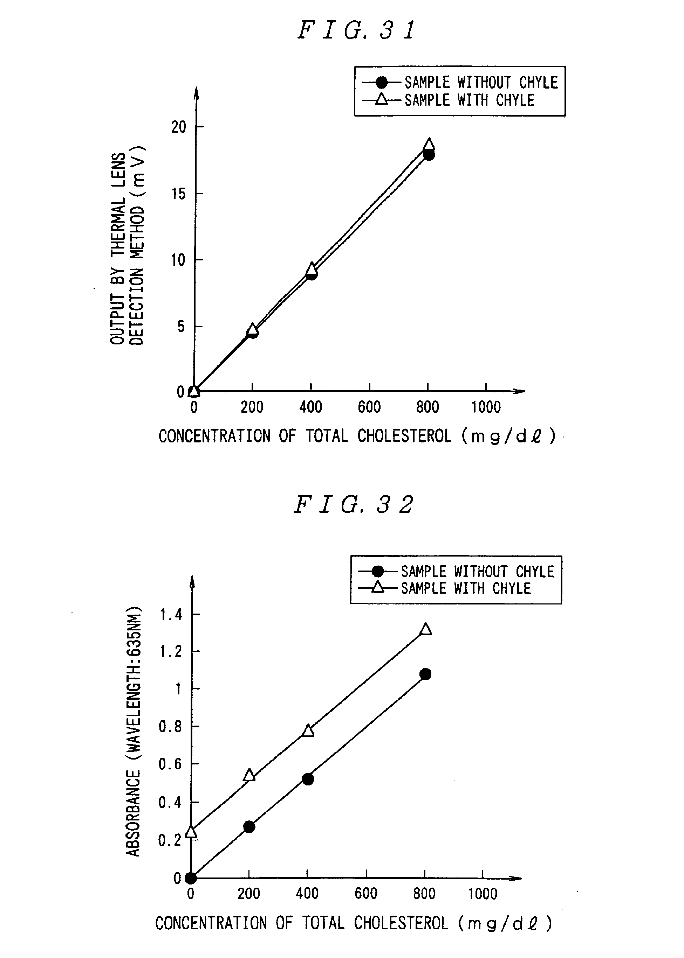

Figure 31 is a graph showing the results of measurements of total cholesterol concentration in a sample having chyle made in accordance with the photothermal converting detection method; and
Figure 32 is a graph showing the results of measurements of total cholesterol concentration in a sample having chyle made in accordance with the absorption spectrophotometry.

### Best Mode for Carrying Out the Invention

### (Example 1)

In measurement of hemoglobin by a thermal lens detection method using a He-Ne laser as excitation light, linearity of relation between the degree of dilution of blood samples and the output by the thermal lens detection method was examined. This will be described below.

The configuration of a photothermal converting detection apparatus is as follows. For a microscope, an inverted microscope (IX70 manufactured by Olympus Optical Co., Ltd.) was used in view of ease of handling of sample of measurements on the stage. An emission microscope may also be used. This microscope is modified so that laser light made to be coaxial by an optical system outside the microscope can be introduced.

As for lasers, a He-Ne laser (633 nm, 10 mW; manufactured by Edmont Scientific Co., Ltd.) was used for excitation, and a semiconductor laser (780 nm; LDT 783 manufactured by Nippon Kagaku Engineering Co., Ltd.) was used for detection (probe light) . For all optical systems such as a mirror and a beam expander, products manufactured by Mesglyo Co., Ltd. were used.

After being modulated by a light chopper, the laser light for excitation is made to be coaxial with the laser light for detection by a dichroic mirror, and is then guided into the microscope to be applied to the sample of measurement. After the laser light for excitation is applied to the sample of measurement, only excitation light out of the laser light made to be coaxial is selectively removed by a filter and guided into a photo sensor. For an element of laser light receiving portion, a photo sensor amplifier with fibers (C6386 manufactured by Hamamatsu Photonics Co., Ltd.) was used in consideration with ease of handling. This photo sensor light receiving portion is wrapped with a cover having pinholes. Outputs from the photo sensor and the sensor amplifier are amplified with a low-noise preamplifier (LI-75A manufactured by N F Circuit Block Co., Ltd.), and are thereafter guided into a lock-in amplifier to be subjected to signal processing.

Using this thermal lens detection apparatus, the relation between the degree of dilution of blood samples and quantifiability of hemoglobin was examined in the following manner.

Blood from healthy subjects with heparin added therein (the concentration of hemoglobin: 16 g/dl) was used as blood samples. For the measurement reagent, a solution (pH 6.0) with potassium ferricyanide, MES and Triton X-100 dissolved therein in the concentrations of 8 mM, 10 mM and 0.8% by weight, respectively, was used.

When the concentration of hemoglobin (Hb) in the sample of measurement was 6400 µg/ml, 960 µl of the above described measurement reagent was added to and mixed with 40 µl of blood sample (mixture ratio: 1:24), and was left standing at room temperature for five minutes or longer, followed by carrying out a measurement by the thermal lens detection method. 996 µl of measurement reagent was added to and mixed with 4 µl of blood sample when the concentration of Hb in the sample of measurement was 640 µg/ml, and 996 µl of measurement reagent was added to and mixed with 4 µl of blood sample diluted to 60% with PBS when the concentration of Hb in the sample of measurement was 384 µg/ml, and 996 µl of measurement reagent was added to and mixed with 4 µl of blood sample diluted to 20% with PBS when the concentration of Hb in the sample of measurement was 128 µg/ml.

Each sample of measurement obtained in this way was put onto a plane glass cuvette having grooves with the depth of 50 µm and wide of several millimeters (Quartz Cell Type AB 20-SQ-0.05 manufactured by GL Science Co., Ltd.), and this cuvette was placed on the stage of the inverted microscope. For this cuvette, as a model version of microchips having complicated groove patterns, a cuvette wide but almost as deep as those of the microchip was adopted. In the thermal lens detection method, the wide of the groove needs to be a few tens of µm or larger, and even if the groove is further widened, there will be no significant problem (however, it may have an influence on diffusion mixing in the groove, etc.). In microchips made of resin and the like, the depth of the groove is limited in terms of production of matrixes for molds, and has a significant influence on sensitivity of the thermal lens detection method. Therefore, the depth of the groove of the plane glass cuvette used in this Example was made to be almost same as that of the microchip described later.

For focusing of objective lenses, the exciting laser was used. In other words, focusing was performed at the rim of the groove of the glass cuvette, referring to the monitor screen. And, thereafter, thermal lens detection was carried out almost at the center of the groove.

The exciting laser was modulated to 3289 Hz by the light chopper to excite methohemoglobin to generate heat. The frequency of this modulation by the light chopper may be changed depending on the influence of S/N ratios and the like. A thermal lens produced by this heat generation shifts the focusing position of the detecting laser, and thereby the amount of light received by the photo sensor through pinholes is changed depending on the amount of generated heat. Signals from the photo sensor are processed with the lock-in amplifier, but in this case, one second was used as a time constant, and only signals having a frequency of 3289 Hz that is same as that of the light chopper were used.

The results are shown in Figure 8. The longitudinal axis represents intensities of outputs (mV) with the thermal lens detection method, the upper stage of the lateral axis represents the concentration of hemoglobin in the blood sample, and the lower stage of the lateral axis represents the concentration of hemoglobin in the sample of measurement after the measurement reagent is mixed.

### (Example 2)

Using a microchip made of organic polymer, measurements of hemoglobin in the blood sample were carried out using a semiconductor laser of 635 nm long as an extiging light. This will be described below.

A plane member having grooves on the surface that constitute a microchip was formed through injection molding. Resin used in the injection molding is methacryl resin (Derpet 80NH manufactured by Asahi Kasei Industry Co., Ltd.). For gas, carbon dioxide with purity of 99% or higher was used, and its pressure was set at 1 MPa. For the molding machine, SG 50 manufactured by Sumitomo Juki Co., Ltd. was used. The molded object is a plane member with the thickness of 2 mm, the length of 120 mm and the width of 60 mm.

A linear groove with the width of 100 µm, the depth of 50 µm and the length of 3 cm is provided on the surface of this plane member. And, at the both ends of this groove, circular grooves (with the diameter of 3 mm and the depth of 50 µm) to provide through-holes for letting in and out sample of measurements are provided. A through-hole with the diameter of 2 mm is bore in this circular groove by drilling or the like to provide a portion for letting in and out sample of measurements.

Furthermore, for matrixes for molds, those obtained by etching silicone with the RIE method and those obtained by photo-curing silicone with DFR (Dry Film Resist) can be used. When after the matrixes were electrocast to prepare stampers, plane members are formed through injection molding, and these members were compared for both matrixes, almost same molded objects were obtained from both the matrixes.

Also, the transferability of the surface condition of the matrix is evaluated by observation using an optical microscope and shape measurement using a laser microscope. Also, the molded object is observed by means of observation using an optical microscope, observation of the shape of the groove in the cut section using an optical microscope and electron microscope, shape measurement using a laser microscope, or the like.

A methacryl resin sheet with thickness of 200 µm coated with photo-curing adhesive was bonded to the groove-bearing surface of the plane member obtained in this way. Then, UV light was applied thereto to form a capillary with only both ends open, and this capillary was used as a microchip for measurement.

For the photothermal converting detection apparatus, the apparatus described in Example 1 was used in principles. However, the laser for excitation light was changed from a He-Ne gas laser of 633 nm to a semiconductor laser with emission of 635 nm (DL-4038-025 manufactured by Sanyo Electric Co., Ltd.), and the optical system was adjusted in association therewith.

For sample of measurements, those obtained by mixing and reacting together blood from healthy subjects with heparin added therein (the concentration of hemoglobin: 16 g/dl) and a measurement regent (a solution (pH 6.0) with potassium ferricyanide, MES and Triton X-100 dissolved therein in the concentrations of 8 mM, 10 mM and 0.8% by weight, respectively) were used.

That is, samples in such a way that 960 µl of the above described measurement reagent was added to and mixed with 40 µl of the above described blood sample when the concentration of hemoglobin in the sample of measurement was 6400 µg/ml, and 996 µl of measurement reagent solution was added to and mixed with 4µl of blood sample with 36 µl of PBS added therein when the concentration of hemoglobin in the sample of measurement was 640 µg/ml, and then they were left standing at room temperature for five minutes or longer, respectively, were used.

2 µl of these sample of measurements (the contents of sample blood was 40 nl and 4 nl) was introduced into a capillary from the through-hole of the microchip prepared as described above to measure outputs by the thermal lens detection method. As a result, output of about 0.5 mV was detected for the hemoglobin concentration of 640 µg/ml, and output of about 5 mV was detected for the hemoglobin concentration of 6400 µg/ml.

### (Example 3)

The case where preparation of the sample of measurement by mixing and reacting together the blood sample and measurement reagent, and measurement of hemoglobin in the sample of measurement by the thermal lens detection method were carried out in a microchip made of organic polymer will now be described.

For the photothermal converting detection apparatus, the same apparatus as that of Example 2 was used.

Also, the microchip was prepared in a same way as Example 2. First, a plane member 31 made of PMMA having grooves on the surface as shown in Figure 9 was formed through injection molding. The outer dimension of the plane member 31 is same as that in Example 2. And, through-holes with the diameter of 1 mm were bore by drilling in circular grooves 1, 2 of this plane member 31 to provide portions for letting in and out blood samples and measurement reagents. Also, similarly, a through-hole was bore in the circular groove 3 to provide a portion for letting out waste liquid.

A methacryl resin sheet with thickness of 200 µm coated with photo-curing adhesive was bonded to surface having grooves having through-holes of the plane member 31 obtained in this way. Then, UV light was applied thereto to form a capillary with only both ends open, and this capillary was used as a microchip for measurement.

Figure 9 shows the channel geometry of the formed capillary. A blood sample reservoir 1, a measurement reagent reservoir 2 and a waste liquid reservoir 3 are provided at the ends of the channels, and for the dimensions of the both reservoirs 1, 2 and the waste liquid reservoir 3 excluding the through-hole portions thereof, the diameter is 3 mm and the depth is 50 µm.

All the capillary (channels) has the depth of 50 µm and the width of 150 µm, and the length is 9 mm for the channel 11, 9 mm for the channel 12 and 65 mm for the channel 13. A detection portion 21 by the photothermal converting detection method (thermal lens detection method) is provided in the channel 13, the distance between a confluence 22 of the channel 11 and channel 12 and the detection portion 21 is 60 mm.

For the measurement reagent, a solution (pH 6.0) with potassium ferricyanide, MES and Triton X-100 dissolved therein in the concentrations of 8 mM, 10 mM and 0.8% by weight, respectively, was used. For the blood sample, blood from healthy subjects with heparin added therein (the concentration of hemoglobin: 16 g/dl) and the above blood sample with the hemoglobin concentration adjusted to 10 g/dl by means of dilution with PBS were used.

4 µl of blood sample was taken in a 25 µl micro syringe (manufactured by Hamilton Co., Ltd.), and the micro syringe was linked to the through-hole of the blood sample reservoir 1 with a Teflon tube. Also, 20 µl of measurement reagent was taken in another micro syringe, and the micro syringe was linked to the through-hole of the measurement reagent reservoir 2 with another Teflon tube.

Both the micro syringes were fitted to two micro syringe pumps (manufactured by Harvard Co., Ltd.) separately, and each of the micro syringe pumps is set so that the flow rate of the blood sample was 0.2 µl/hr and the flow rate of the measurement reagent was 4.8 µl/hr. Consequently, the mixture ratio of the blood sample and the measurement reagent would be 1:24. Reaction time after mixing them equals a time period over which they flow through the channel 13 of Figure 9, which would be about 5.4 minutes.

Both the above described micro syringe pumps were driven at room temperature to measure outputs by the thermal lens detection method. As a result, output of about 4.0 mV was obtained for the blood sample with the hemoglobin concentration of 16 g/dl, and output of about 2.5 mV was obtained for the diluted blood sample with the hemoglobin concentration adjusted to 10 g/dl.

### (Reference Example 1)

Measurements of hemoglobin were carried out in a same way as Example 1 except that an Ar laser having emission at 488 nm was used as a source of probe light.

For blood samples, blood from two healthy subjects with heparin added therein (the concentration of hemoglobin: 16 g/dl) was used as samples 1 and 2. These blood samples were diluted with PBS so that their concentrations were 60% and 20%, and the diluted samples were defined as the sample 0.6 × sample and 0.2 × sample. 996 µl of measurement reagent (a solution (pH 6.0) with potassium ferricyanide, MES and Triton X-100 dissolved therein in the concentrations of 0.61 mM, 10 mM and 0.8% by weight, respectively) was added to and mixed with 4 µl of these diluted samples or non-diluted blood sample (1 x sample), and was left standing at room temperature for five minutes or longer, followed by carrying out measurements by the thermal lens detection method.

The results are shown in Table 1. Furthermore, the values in Table 1 represent outputs by the thermal lens detection method after subtracting backgrounds.

As apparent from the results, outputs by the thermal detection method were not stable in general, and in particular, abnormally high values were shown for diluted samples. These phenomena have not found in the aforesaid Examples.

As a cause of such phenomena, it can be considered that due to the fact that excitation light of 633 nm and probe light of 488 nm are applied at the same time, a variety of excited states of hemoglobin are aroused to produce photoreaction like exciplex, or other components in the blood absorb the light of 488 nm to interact with hemoglobin. However, details thereon have not been clarified yet.

**(Table 1)**

| | Sample 1 | Sample 2 |
|---|---|---|
| Diluted to 20% | 200 to 300 mV | Increased from 3 mV to 250 mV in 10 minutes |
| Diluted to 60% | 2 to 6 mV | 3 to 6 mV Gradually Increased |
| Not diluted | 6 mV Unstable | Stable at 3.1 mV |

### (Example 4)

The result of quantitatingALP activity in the blood serum by the photothermal converting detection method is shown as one example of the present invention.

### (Configuration of photothermal Converting Detection System)

For the photothermal converting detection system (photothermal converting detection apparatus), a system identical in basic configuration to that of Example 1 was used. General procedures of measurements by the photothermal converting detection system in the measurement of substances other than hemoglobin are as follows.

A plane microchip having a groove pattern (or a glass cell containing a sample of measurement. The depth of the glass cell is 50, 100, 200 and 500 µm) is placed on the stage of an inverted microscope. For the focusing of the objective lens, an exciting laser was used to perform focusing at the positions of the upper and lower edges of the groove pattern referring to the monitor screen, followed by setting the middle point thereof to be the central position of the groove.

After focusing is performed, reaction between the sample and measurement reagent as described above is carried out to guide the solution containing the reaction product to the detection portion. The exciting laser is modulated to 1170 Hz by the light chopper to excite the reaction product in the groove to generate heat. The frequency of this modulation by the light chopper may be changed depending influences of S/N ratios and the like.

A thermal lens produced by this heat generation shifts the focusing position of the detecting laser, and thereby the amount of light received by the photo sensor through pinholes is changed depending on the amount of generated heat. The sample of measurement may be flown or stop being flown during measurement, but in this Example, measurements were carried out in the state in which the sample stopped being flown. Signals from the photo sensor are processed by the lock-in amplifier, but in this case, one second was used as a time constant, and only signals having a frequency of 1170 Hz that was same as that of the light chopper were selectively used. Because the output voltage of the lock-in amplifier is proportional to the concentration of the reaction product excited by excitation light, the reaction product can be quantitated.

### (Preparation of Measurement reagent)

In accordance with JSCC Standard Method, a buffer 1 was prepared in the same way as described above. Then, BCIP (Wako Pure Chemical Industries, Ltd.) was dissolved as a substrate in the buffer 1 so that 30, 15 and 7 mM were obtained as final concentrations to prepare an ALP measurement reagent (measurement reagent),immediately before the measurement reagent was reacted with the blood sample.

### (Sample: Preparation of Standard Serum)

For the sample, Switrol A (Nissui Pharmaceutical Co., Ltd.) of which preparation process was partially changed was used. Specifically, a vial of freeze-dried product was dissolved in 2500 µl of purified water (Kyoei Pharmaceutical Co., Ltd.) so that the ALP activity was 302 IU/l as a calculated value to prepare a stock solution. Then, the stock solution was diluted with purified water to prepare solutions having ALT activities of 101 IU/l and 51 IU/l as calculated values. And, those solutions and purified water itself were adopted as samples.

### (Detection of ALP Activity)

1000 µl of ALP measurement reagent and 25 µl of sample are fully mixed together in a test tube, and are thermally insulated at 37°C for accurate 2.5 minutes. Thereafter, the mixed solution (sample of measurement) is put onto a plane glass cuvette having 50 µm deep grooves (Quartz Cell Type AB20-SQ-0.05 manufactured by GL Sciences Co., Ltd.), and this cuvette is placed on the stage of an inverted microscope with an air thermostat of 37°C attached thereto. The focusing of the objective lens is carried out seeing output values of the lock-in amplifier, with the position showing the highest value as an indicator.

Outputs by the thermal lens detection method for about 15 minutes after start of measurement were processed with the aforesaid software LabVIEW. That is, the obtained curve was subjected to linear regression, and quantifications were performed by defining the slope of the line formed through regression as the amount of produced reaction per second. In the meantime, the cuvette was kept at 37°C by air thermal isolation on the stage of the microscope. The results obtained from the above described measurements are shown in Figure 10.

### (Comparative Example 1)

As one comparative example of the present invention, the case where measurements by the thermal lens method were difficult to carry out due to occurrence of precipitation of insoluble substrate (BCI) is shown.

### (Configuration of Photothermal Converting detection System)

The photothermal converting detection system has a configuration similar to that of Example 4.

### (Preparation of Measurement reagent)

The reagent was prepared in a same way as Example 4. However, the BCIP concentration was 70 mM.

### (Preparation of Sample)

The sample was prepared in a same way as Example 4. However, the ALP activity was 800 IU/l.

### (Detection of ALP Activity)

The detection was carried out in a same way as Example 4. As a result, for the ALP measurement reagent with the BCIP concentration of 70 mM, stable outputs by the thermal lens detection method could not be obtained, and thus the reacted amount could not be determined, as shown in Figure 11. In contrast to this, for the ALP measurement reagent with the BCIP concentration of 15 mM, outputs by the thermal lens detection method with the gradient stabilized over time could be obtained.

Also, as a result of keeping the ALP measurement reagent with the BCIP concentration of 70 mM in cooled conditions at 4°C, recrystallization of BCIP was found. This phenomenon was also found for the ALP measurement reagent with the BCIP concentration of 35 mM, but was not found for the ALP reagent with the BCIP concentration of 15 mM or lower.

From this, it could be considered that instability of outputs by the thermal lens detection method was due to the fact that colored fine crystals were formed and the fine crystals (fine particles) caused scattering.

### (Comparative Example 2)

As one comparative example, the case where the measurement of absorbance is possible even when fine crystals of insoluble substrate (BCI) are formed will be described. For the ALP measurement reagent and sample, those prepared in a same way as Example 4 were used. For the spectrophotometer, UV 2200 manufactured by Shimadzu Corp. was used. As a result, a calibration curve could be obtained even when the ALP measurement reagent with the BCIP concentration of 70 mM, as shown in Figure 12.

### (Example 5)

A microchip made of resin with capillaries (grooves) provided therein was used to quantitate ALP activity while the flow rates of the ALP measurement reagent and sample are controlled. Liquid delivery and the flow rate were controlled mechanicallybydrivingmicro syringes linked to the reservoirs through pipes using a syringe pump and micro peristaltic pump made by modifying the syringe pump that were connected in series to each other.

### (Preparation of Microchip)

The microchip was prepared in an almost same way as Example 3. The formed plane member 31 is 120 mm long and 80 mm wide and 5 mm thick, and has grooves provided thereon forming a pattern similar to that of the microchip shown in Figure 9.

Through-holes with the diameter of 1.5 mm are bore in the liquid reservoirs 1 to 3 by drilling. The liquid reservoir 1 is for the sample, the liquid reservoir 2 is for the measurement reagent, and the liquid reservoir 3 is for the waste liquid. Furthermore, if two types of measurement reagents are used to carry out measurements, a microchip having a pattern further comprising a liquid reservoir 4 at an appropriate point between the confluence 22 of the groove 13 and the detection portion 21 is used.

All the grooves have the depth of 50 µm, and for the width and length of grooves, the groove 11 has the width of 50 µm and the length of 5 mm, the groove 12 has the width of 50 µm and the length of 5 mm, and the groove 13 has the width of 100 µm and the length of 9 mm. A methacryl resin sheet with the thickness of 300 µm was bonded to the surface of this plane member 31 having grooves using an acryl based light curing adhesive dissolved in methaacrylate monomer.

Then, a disposable syringe needle was cut at a position about 3 mm from the basal portion to remove the tip, thereby preparing an external conical liquid reservoir. This external liquid reservoir had an internal diameter of 4 mm and height of 10 mm. Three external liquid reservoirs were bonded to the plane member 31 in such amanner that the reservoirs communicated with the liquid reservoirs 1 to 3, respectively, to complete the microchip.

### (Configuration of Liquid Delivery System)

A liquid delivery system comprising micro syringes 41, a syringe pump 40, and a peristaltic pump 50 made by modifying the syringe pump are shown in Figure 13. Also, a systematic diagram illustrating the configuration of the peristaltic pump 50 is shown in Figure 14.

The tips of the micro syringes 41, 41 (manufactured by Hamilton Co., Ltd., capacity of 500 µl) attached to the syringe pump 40 (manufactured by Harvard Co., Ltd.) are connected to the one ends of peristaltic tubes 51, 51 (internal diameter of 250 µm) attached to the peristaltic pump 50 through ethylene vinyl acetate tubes 42, 42 (internal diameter of 350 µm). And, the other ends of the peristaltic tubes 51, 51 are connected to two external liquid reservoirs 61, 61 communicating with the liquid reservoirs 1, 2 of the microchip 60 through SUS tubes 52, 52.

In the peristaltic pump 50, the peristaltic tube 51 fixed to the peristaltic pump 50 by a tube stopper 55 is sandwiched between a roller 53 and an aluminum plane plate 54. And, the roller 53 moves in the longitudinal direction along the peristaltic tube 51, whereby liquid in the peristaltic tube 51 is pushed toward the polymer chip 60.

Owing to such a configuration, theALPmeasurement reagent and sample are delivered from the micro syringe 41 to the microchip 60 with their flow rates being controlled.

### (Configuration of Photothermal Converting Detection System)

For the photothermal converting detection system (photothermal converting detection apparatus), a system identical in basic configuration to that of Example 1 was used. However, the laser for excitation light was changed from a He-Ne gas laser of 633 nm to a semiconductor laser with emission of 635 nm (DL-4038-025 manufactured by Sanyo Electric Co., Ltd.). And the optical system was adjusted in association with this change.

### (Preparation of Measurement reagent)

The reagent was prepared in a same way as Example 4. However, the BCIP concentration was 15 mM.

### (Sample: Preparation of Standard Serum)

For the sample, Switrol A (Nissui Pharmaceutical Co., Ltd.) of which preparation process was partially changed was used. Specifically, a vial of freeze-dried product was dissolved in 2500 µl of purified water (Kyoei Pharmaceutical Co., Ltd.) so that the ALP activity was 350 IU/l as a calculated value to prepare a stock solution. Then, the stock solution was diluted with purified water to prepare solutions having ALT activities of 175 IU/l and 100 IU/l as calculated values. Furthermore, 20 µl of the above described two solutions and 20 µl of purified water were mixed with 480 µl of buffer 1, and the resulting solutions were adopted as samples. A small amount (0.1 vol %) of suspension of latex beads with the diameter of 0.5 µm (Cell Monitor Particles For Plane Samples manufacturedby Otsuka Electronic Co., Ltd.) for checking flows was added in each solution.

### (Detection of ALP Activity)

Using a syringe or the like, the measurement reagent was injected into the liquid reservoir 1 and the sample was injected into the liquid reservoir 2 manually. The microchip 60 containing both the liquids was placed on the stage of the inverted microscope with an air thermostat of 37°C attached thereto.

Then, the liquid reservoirs 1 and 2 of the microchip 60 were connected to the peristaltic tube 51 attached to the peristaltic pump 50 through the SUS tube to complete a liquid delivery system having a configuration as shown in Figure 13.

By driving the syringe pump 41, the measurement reagent and sample were flowed at the flow rate of 600 µl/h for about three minutes to introduce the above described both liquids into the capillary of the microchip 60. Thereafter, the syringe pump 41 was stopped, and ALP activity was measured in a same way as Example 4. As a result, a calibration curve as shown in Figure 15 was obtained.

### (Example 6)

### (Measurement of Particle Size Distribution)

Fine crystals may exist in the sample of measurement obtained by mixing the ALP measurement reagent with the sample (sample originating in biological components) . The particle size distribution of such fine crystals was measured, and the results thereof will be described.

Samples (Samples 1 to 4) obtained by mixing 25 ml of ALP measurement reagents with the BCIP concentrations of 70 mM and 6.25 mM and standard serum (samples) prepared so that the ALP activity was 800 IU/l as a calculated value in the following manner were prepared, respectively.

First, Sample 1 is a sample obtained by mixing 2500 µl of standard serum with the ALP measurement reagent with the BCIP concentration of 70 mM and allowing the mixture to react at 37°C for 3 days.

Then, Sample 2 is a sample obtained by mixing 625 µl of standard serum with the ALP measurement reagent with the BCIP concentration of 70 mM and allowing the mixture to react at 37°C for 3 days.

Further, Sample 3 is a sample obtained by mixing 625 µl of standard serum with the ALP measurement reagent with the BCIP concentration of 6.25 mM and allowing the mixture to react at 37°C for 3 hours.

Further, Sample 4 is only the ALP measurement reagent with the BCIP concentration of 6.25 mM (the standard serum was not mixed therewith).

HORIBA LA-910 Laser Diffraction / Dispersion Type Particle Size Distribution Measuring Apparatus was used as a measuring apparatus to carry out measurements of particle size distribution. Specifically, 10 ml of Samples 1 to 4 were added in an ultrasonic dispersion bath attached to the above described measuring apparatus containing about 50 ml of purified water to carry out the measurement. The relative refractive index as a measurement condition of the particle size distribution was set at 1.26 to 0.00 i.

The results of the measurement are shown in Figure 16. In the measurement reagent obtained by mixing the ALP measurement reagent and the standard serum together and allowing the mixture to react, BCI2 produced as a result of the above described reaction exists. And, particles (fine crystals) of the BCI2 have been found to have a diameter of about a few hundred nm.

Also, it has been apparent from the result of the measurement of particle size distribution that particles with the size of a several µm also exist. However, because particles with the size of a few hundred nm are predominant in the case of samples obtained after long hours of reaction, namely Samples 1 and 2 obtained after three days of reaction, it can be considered that particles with the size of several µm are of BCIP. However, the state in which particles of BCIP exist has not been clarified.

Therefore, for a measurement reagent suitable for the thermal lens detection method, pigment particles should not be produced as products of reaction between the measurement reagent and the sample originating in biological components. In addition, the BCIP concentration of the ALP measurement reagent and reaction time should be appropriate.

Furthermore, needles to say, it is not preferable that fine crystals such as particles of BCI2 exist before the reaction between the measurement reagent and the sample originating in biological components.

### (Example 7)

As one example of the present invention, the result of quantitating LDH activity in the blood serum will be described. Furthermore, the measurement is carried out in a microchip made of resin by the photothermal converting detection method.

### (Configuration of Photothermal Converting Detection System)

For the photothermal converting detection system (photothermal converting detection apparatus), a system identical in basic configuration to that of Example 1 was used. However, the laser for excitation light was changed from a He-Ne gas laser of 633 nm to a semiconductor laser with emission of 635 nm (DL-4038-025 manufactured by Sanyo Electric Co., Ltd.). And the optical system was adjusted in association with the change.

### (Preparation of Measurement reagent)

LDH Kainos (manufactured by Kainos CO., Ltd.) being a measurement reagent kit was used to prepare a measurement reagent.

That is, 2,2'-Dibenzothiazolyl-5,5'-bis [4-di(2-sulfoethyl) carbamoylphenyl] -3,3'-(3,3'-dimethoxy-4,4'-bisphenylen) ditetrazolium disodium salt (WST-5 manufactured by Dojin Laboratories) was added to the LDH Kainos from which NTB being a coloring pigment had been removed. More specifically, 0.5 mM WST-5 was dissolved in 16 ml of LDH Kainos (substrate buffer) from which NTB had been removed, and then the reaction reagent was dissolved therein to provide a measurement reagent.

### (Sample: Preparation of Standard Serum)

For the sample, Switrol A (Nissui Pharmaceutical Co., Ltd.) of which preparation process was partially changed was used. Specifically, a vial of freeze-dried product was dissolved in 5 ml of purified water (Kyoei Pharmaceutical Co., Ltd.) so that the LDH activity was 700 WU as a calculated value to prepare a stock solution. Then, the stock solution was diluted with purified water to prepare solutions having LDH activities of 400 WU and 100 WU as calculated values. And, these solutions and purified water itself were adopted as sample.

### (Detection of LDH Activity)

Measurements of LDH activity were carried out in a same way as Example 5. As a result, a calibration curve as shown in Figure 17 was obtained.

### (Example 8)

### (Measurement of TP)

For the measurement reagent, Kainos Auto Series TP Reagent (Kainos Co., Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the TP concentrations were 9.75 g/dl, 7.5 g/dl and 5.0 g/dl as calculated values. And, these solutions and purified water itself were adopted as samples.

For the photothermal converting detection apparatus, an apparatus having a configuration similar to that of Example 4 was used. And, the above described sample and the above described measurement reagent were mixed and reacted together in accordance with the specifications of the above described measurement reagent. Thereafter, the sample of measurement was put onto a plane glass cuvette having 50 µm deep grooves (Quartz Cell Type AB20-SQ-0.05 manufactured by GL Sciences Co., Ltd.), and this cuvette was placed on the stage of an inverted microscope. The focusing of the objective lens is carried out seeing output values of the lock-in amplifier, with the position showing the highest value as an indicator. This highest value was processed with the aforesaid software LabVIEW to measure the TP concentration. As a result, a calibration curb as shown in Figure 18 was obtained.

### (Example 9)

### (Measurement of Urea Nitrogen)

For the measurement reagent, Reagent for Autoanalysis "Seiken" UN-I (Denka Seiken Co., Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of urea nitrogen were 99.4 mg/dl, 24.9 mg/dl and 6.2 mg/dl as calculated values. And, these solutions and the purified water itself were adopted as samples.

For the photothermal converting detection apparatus, an apparatus having a configuration similar to that of Example 4 was used. And, the concentration of urea nitrogen was measured in a same way as Example 8. As a result, a calibration curve as shown in Figure 19 was obtained.

### (Example 10)

### (Measurement of Total Bilirubin)

For the measurement reagent, HA Test Wako Total Bilirubin II-HA Test Wako for Measurement of Total Bilirubin (Wako Pure Chemical Industries, Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of total bilirubin were 1.68 mg/dl, 0.4 mg/dl and 0.1 mg/dl as calculated values. And, these solutions and the purified water itself were adopted as samples.

For the photothermal converting detection apparatus, an apparatus having a configuration similar to that of Example 4 was used. And, the concentration of total bilirubin was measured in a same way as Example 8. As a result, a calibration curve as shown in Figure 20 was obtained.

### (Example 11)

### (Measurement of γ-GTP Activity)

For the measurement reagent, γ-GTP Kainos (Kainos Co., Ltd.) was used in accordance with its specifications.

For the sample,Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that γ-GTP activities were 121I U/l, 80 IU/l and 40 IU/l as calculated values. And, these solutions and the purified water itself were adopted as samples.

For the photothermal converting detection apparatus, an apparatus having a configuration similar to that of Example 4 was used. And, the γ-GTP activity was measured in a same way as Example 8. As a result, a calibration curve as shown in Figure 21 was obtained.

### (Example 12)

### (Measurement of Albumin)

For the measurement reagent, Albumin Reagent/A (Kokusai Siyaku Co., Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of albumin were 7.0 g/dl, 4.71 g/dl and 2.35 g/dl as calculated values. And, these solutions and the purified water itself were adopted as samples.

For the photothermal converting detection apparatus, an apparatus having a configuration similar to that of Example 4 was used. And, the concentration of albumin was measured in a same way as Example 8. As a result, a calibration curve as shown in Figure 22 was obtained.

Examples of measurements for a variety of substances will be described as a reference in the following the Reference Examples 2 to 9.

### (Reference Example 2)

### (Measurement of AST Activity)

For the measurement reagent, TA-LN Kainos (Kainos Co., Ltd.) was used in accordance with its specifications. However, this measurement reagent contains DAOS (Dojin Laboratories) instead of TOOS contained as usual in TA-LN Kainos. Its concentration is identical to that of the reagent containing TOOS as usual.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that AST activities were 238 KU, 33.6 KU and 1.7 KU as calculated values. And, these solutions and the purified water itself were adopted as samples.

For the photothermal converting detection apparatus, an apparatus having a configuration similar to that of Example 4 was used. And, the AST activity was measured in a same way as Example 4. As a result, a calibration curve as shown in Figure 23 was obtained.

### (Reference Example 3)

### (Measurement of Uric Acid)

For the measurement reagent, Iatro LQ UA (Yatron Co., Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of uric acid were 9.79 mg/dl, 6.53 mg/dl and 3.25 mg/dl as calculated values. And, these solutions and the purified water itself were adopted as samples.

For the photothermal converting detection apparatus, an apparatus having a configuration similar to that of Example 4 was used. And, the concentration of uric acid was measured in a same way as Example 8. As a result, a calibration curve as shown in Figure 24 was obtained.

### (Reference Example 4)

### (Measurement of Glucose)

For the measurement reagent, Determiner GL·E (KyowaMedix Co., Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of glucose were 305 mg/dl, 101.7 mg/dl and 50.9 mg/dl as calculated values. And, these solutions and the purified water itself were adopted as samples.

For the photothermal converting detection apparatus, an apparatus having a configuration similar to that of Example 4 was used. And, the concentration of glucose was measured in a same way as Example 8. As a result, a calibration curve as shown in Figure 25 was obtained.

### (Reference Example 5)

### (Measurement of Total Cholesterol)

For the measurement reagent, HA Test Wako Pure Cholesterol E-HA Test Wako for Measurement of Total Cholesterol (Wako Pure Chemicals Industries, Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of total cholesterol were 800 mg/dl, 400 mg/dl and 200 mg/dl as calculated values. And, these solutions and the purified water itself were adopted as samples.

For the photothermal converting detection apparatus, an apparatus having a configuration similar to that of Example 4 was used. And, the concentration of total cholesterol was measured in a same way as Example 8. As a result, a calibration curve as shown in Figure 26 was obtained.

### (Reference Example 6)

### (Measurement of HDL-Cholesterol)

For the measurement reagent, Chores Test HDL (Daiichi Pure Chemicals Co., Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of HDL-cholesterol were 76.8 mg/dl, 48 mg/dl and 24 mg/dl as calculated values. And, these solutions and the purified water itself were adopted as samples.

For the photothermal converting detection apparatus, an apparatus having a configuration similar to that of Example 4 was used. And, the concentration of HDL-cholesterol was measured in a same way as Example 8. As a result, a calibration curve as shown in Figure 27 was obtained.

### (Reference Example 7)

### (Measurement of LDL-Cholesterol)

For the measurement reagent, Chores Test LDL (Daiichi Pure Chemicals Co., Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of LDL-cholesterol were 270 mg/dl, 135 mg/dl and 62.5 mg/dl as calculated values. And, these solutions and the purified water itself were adopted as samples.

For the photothermal converting detection apparatus, an apparatus having a configuration similar to that of Example 4 was used. And, the concentration of LDL-cholesterol was measured in a same way as Example 8. As a result, a calibration curve as shown in Figure 28 was obtained.

### (Reference Example 8)

### (Measurement of Neutral Fat)

For the measurement reagent, Reagent for Autoanalyzer-HR Triglyceride-HR (Wako Pure Chemical Industries, Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of neutral fat were 208 mg/dl, 104 mg/dl and 34.7 mg/dl as calculated values. And, these solutions and the purified water itself were adopted as samples.

For the photothermal converting detection apparatus, an apparatus having a configuration similar to that of Example 4 was used. And, the concentration of neutral fat was measured in a same way as Example 8. As a result, a calibration curve as shown in Figure 29 was obtained.

### (Reference Example 9)

### (Measurement of Creatinine)

For the measurement reagent, L Type Creatinine F for Measurement of Creatinine (Wako Pure Chemical Industries, Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of creatinine were 1.58 mg/dl, 0.79 mg/dl and 0.39 mg/dl as calculated values. And, these solutions and the purified water itself were adopted as samples.

For the photothermal converting detection apparatus, an apparatus having a configuration similar to that of Example 4 was used. And, the concentration of creatinine was measured in a same way as Example 8. As a result, a calibration curve as shown in Figure 30 was obtained.

The results of measurements of blood samples with chyle will now be described.

### (Example 13)

For the measurement reagent, HA Test Wako Cholesterol E-HA Test Wako (Wako Pure Chemical Industries, Ltd.) was used in accordance with its specifications.

For the sample, Switrol A (Nissui Pharmaceutical Co., Ltd.) of which preparation process was partially changed was used. Specifically, a vial of freeze-dried product was dissolved in purified water (Kyoei Pharmaceutical Co., Ltd.) so that the concentrations of total cholesterol were 800 mg/dl, 400 mg/dl, 200 mg/dl and 0 mg/dl (purified water itself) as calculated values. And, solutions obtained by diluting these solutions with Chyle and Chyle Blank of Interference Check / A Plus (Kokusai Siyaku Co., Ltd.) so that their concentrations were reduced by a factor of ten were adopted as samples. Furthermore, solutions diluted with Chyle of Interference Check / A Plus are samples with chyle, and solutions diluted with Chyle Blank of Interference Check / A Plus are samples without chyle.

The sample with chyle had a formazine level of 2100 degrees after dilution.

For the photothermal converting detection apparatus, an apparatus having a configuration similar to that of Example 4 was used. And, the concentration of total cholesterol was measured in a same way as Example 8. As a result, a calibration curve as shown in Figure 31 was obtained. As apparent from the graph of Figure 31, the calibration curve of the sample with chyle is almost identical to the calibration curve of the sample without chyle. From this result, it can be understood that in the photothermal converting detection method, there is little influence of chyle.

Then, for a sample of measurement prepared in a similar way, measurements of absorbance at the wavelength 635 nm were carried out using a spectrophotometer (Shimadzu UV 2200). The result thereof is shown in Figure 32.

From this result, it has been understood that the absorbance of the sample with chyle is about 0.2 larger than that of the sample without chyle. From this fact, it can be understood that in the measurement of absorbance, it is necessary to measure absorbance at two different wavelengths for subtracting the background.

### Industrial Applicability

As described so far, according to the method for measuring substances of this invention, the quantity of substances, such as hemoglobin andALP, in a very small amount of sample obtained from the constituent of living organism can be measured simply and easily and for a short period of time in accordance with the photothermal converting detection method. Further, according to the method for measuring substances of this invention, measurements can be carried out stably, without causing precipitates or crystallites of the reaction product in a capillary, in accordance with the photothermal converting detectionmethod. Since the method uses, as excitation light, laser light which outputs light having a long wavelength, the photothermal converting detection apparatus can be manufactured inexpensively, and consequently, measurements can be carried out at low costs.

Further, according to the method for measuring substances of this invention, a sample preparation step and a photothermal conversion detection step are carried out in a microchip; therefore, only a very small amount of sample obtained from the constituent of living organism as well as measurement reagent is required for measurements. Further, the operation for preparing a sample for measurements by mixing a sample obtained from the constituent of living organism and a measurement reagent is simple and easy, and moreover, the amount of wastes produced is small.

According to the method for measuring hemoglobin of this invention in particular, hemoglobin can be measured in a stable manner. In addition, the measurement reagent can be handled with no special risk and troublesome waste disposal is not required, since it contains no cyanide, a poisonous substance.

Further, according to the method for measuring substances of this invention, even if a blood sample has chyle, which is a problem in clinical laboratory tests, in it, it is not necessary to make measurements at two different wavelengths, and quantitative determination can be made at a time.

Still further, the use of the measurement reagent of this invention allows the quantity of substances, such as hemoglobin and ALP, in a very small amount of sample obtained from the constituent of living organism to be measured stably in accordance with the photothermal converting detection method.

## Claims

1. A method for detecting a substance in a sample obtained from a constituent of living organism or measuring a concentration of the same, **characterized in that**
it includes a sample preparation step and a photothermal converting detection step;
the sample preparation step is a step of preparing a sample for measurement in the sub-steps of: mixing and reacting the sample obtained from a constituent of living organism with a measurement reagent; and producing a reaction product, without producing hydrogen peroxide, having an absorption peak at a wavelength different from that of the absorption peak of the substance in an amount corresponding to that of the substance; and
the photothermal converting detection step is a step of measuring a change in physical quantity of the sample for measurement with a change in local temperature of the same which is caused by irradiating the sample for measurement with excitation light,
the reaction product being water-soluble, and the absorbance of the reaction product in a wavelength range of the excitation light being sufficient to cause a temperature change.

2. The method for measuring a substance according to claim 1, **characterized in that** the change in physical quantity is a change in refractive index and the photothermal converting detection step is a step of measuring a change in probe light, which is made to enter a thermal lens formed due to the change in refractive index, caused by the thermal lens.

3. The method for measuring a substance according to claim 1 or 2, **characterized in that** the wavelength of the excitation light is 600 nm or more.

4. The method for measuring a substance according to any one of claims 1 to 3, **characterized in that** the sample preparation step and the photothermal converting detection step are carried out in a capillary of a microchip by charging the microchip including a capillary with the sample obtained from a constituent of living organism and the measurement reagent.

5. The method for measuring a substance according to claim 4, **characterized in that** the capillary is formed by bonding a pair of planar members together, at least one of the pair of planar members includes a groove on its surface, and the pair of the planar members are bonded together with the surface including the groove facing inside.

6. The method for measuring a substance according to any one of claims 1 to 5, **characterized in that** the sample obtained from a constituent of living organism is a blood sample, the substance is hemoglobin and the sample preparation step is a step of preparing the sample for measurement by mixing the blood sample and the measurement reagent to hemolyze the blood sample.

7. The method for measuring a substance according to claim 6, **characterized in that** the wavelength of the excitation light is in the range of 610 nm to 650 nm and the wavelength of the probe light is longer than that of the excitation light.

8. The method for measuring a substance according to claim 6, **characterized in that** the wavelength of the excitation light is in the range of 620 nm to 640 nm and the wavelength of the probe light is longer than that of the excitation light.

9. The method for measuring a substance according to any one of claims 6 to 8, **characterized in that** the measurement reagent contains a neutral surfactant in a concentration sufficient to hemolyze red blood cells, an oxidant in a concentration sufficient to oxidize hemoglobin to give methemoglobin, and a buffer in a concentration sufficient to keep a pH value of the reagent in the range of 5 to 7.

10. The method for measuring a substance according to claim 9, **characterized in that** the measurement reagent contains no cyanide.

11. The method for measuring a substance according to any one of claims 6 to 10, **characterized in that** the mixing ratios of the blood sample to the measurement reagent are in a range of 1:1 to 1:250.

12. The method for measuring a substance according to any one of claims 1 to 5, **characterized in that** the substance is phosphatase, the measurement reagent contains a substrate for the phosphatase, and the sample preparation step is a step of preparing a sample for measurement by mixing the sample obtained from a constituent of living organism with the measurement reagent to react the phosphatase in the sample obtained from a constituent of living organism with the substrate in the measurement reagent.

13. The method for measuring a substance according to claim 12, **characterized in that** the substrate has a phosphoric ester linkage.

14. The method for measuring a substance according to claim 12 or 13, **characterized in that** the substrate is any one of 5-bromo-4-chloro-3-indoxyl phosphate and the salts thereof.

15. The method for measuring a substance according to any one of claims 12 to 14, **characterized in that** the phosphatase is alkaline phosphatase.

16. The method for measuring a substance according to any one of claims 12 to 15, **characterized in that** the concentration of the substrate in a sample for measurement is in a range of 1 to 15 mM.

17. The method for measuring a substance according to any one of claims 12 to 16, **characterized in that** total time from mixing the sample obtained from a constituent of living organism with the measurement reagent to measuring the change in physical quantity is in a range of 3 to 15 minutes.

18. A method for detecting a substance in a blood sample with chyle or measuring a concentration of the same, **characterized in that** it contains a sample preparation step of preparing a sample for measurement by mixing the blood sample with a measurement reagent and a photothermal converting detection step of measuring a change in physical quantity of the sample for measurement with a change in local temperature of the same which is caused by irradiating the sample for measurement with excitation light.

19. The method for measuring a substance according to claim 18, **characterized in that** the change in physical quantity is a change in refractive index and the photothermal converting detection step is a step of measuring a change in probe light, which is made to enter a thermal lens formed due to the change in refractive index, caused by the thermal lens.

20. The method for measuring a substance according to claim 18 or 19, **characterized in that** the sample preparation step and the photothermal converting detection step are carried out in a capillary of a microchip by charging the microchip including a capillary with the blood sample and the measurement reagent.

21. The method for measuring a substance according to claim 20, **characterized in that** the capillary is formed by bonding a pair of planar members together, at least one of the pair of planar members includes a groove on its surface, and the pair of the planar members are bonded together with the surface including the groove facing inside.

22. A measurement reagent used for the method for measuring a substance according to anyone of claims 6 to 11, **characterized in that** it contains a neutral surfactant in a concentration sufficient to hemolyze red blood cells, an oxidant in a concentration sufficient to oxidize hemoglobin to give methemoglobin, and a buffer in a concentration sufficient to keep a pH value of the reagent in a range of 5 to 7.

23. A measurement reagent used for the method for measuring a substance according to any one of claims 12 to 17,
**characterized in that** it contains a substrate having a phosphoric ester linkage.

24. The measurement reagent according to claim 23,
**characterized in that** the substrate is any one of 5-bromo-4-chloro-3-indoxyl phosphate and the salts thereof.
